(19) Europäisches Patentamt
European Patent Office
Office européen des brevets

(11) **EP 2 728 014 B1**

(12) **EUROPEAN PATENT SPECIFICATION**

(45) Date of publication and mention
of the grant of the patent:
**07.10.2015 Bulletin 2015/41**

(51) Int Cl.:
***C12Q 1/68*** *(2006.01)*

(21) Application number: **12190844.6**

(22) Date of filing: **31.10.2012**

(54) **Non-invasive method for detecting a fetal chromosomal aneuploidy**

Nicht invasives Verfahren zum Erfassen einer chromosomalen Fötusaneuploidie

Procédé non invasif permettant de détecter une aneuploïdie chromosomique fétale

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(43) Date of publication of application:
**07.05.2014 Bulletin 2014/19**

(73) Proprietor: **Genesupport SA**
**1700 Fribourg (CH)**

(72) Inventor: **Conrad, Bernard**
**1700 Fribourg (CH)**

(74) Representative: **Rentsch Partner AG**
**Rechtsanwälte und Patentanwälte**
**Fraumünsterstrasse 9**
**Postfach 2441**
**8022 Zürich (CH)**

(56) References cited:
**WO-A1-2011/090556      US-A1- 2012 270 739**

- CHIU ROSSA W K ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES, NATIONAL ACADEMY OF SCIENCES, US, vol. 105, no. 51, 23 December 2008 (2008-12-23), pages 20458-20463, XP002620454, ISSN: 0027-8424, DOI: 10.1073/PNAS.0810641105 [retrieved on 2008-12-10]

- R. W. K. CHIU ET AL: "Non-invasive prenatal assessment of trisomy 21 by multiplexed maternal plasma DNA sequencing: large scale validity study", BMJ, vol. 342, no. jan11 1, 11 January 2011 (2011-01-11), pages c7401-c7401, XP055024134, ISSN: 0959-8138, DOI: 10.1136/bmj.c7401
- ERIC Z. CHEN ET AL: "Noninvasive Prenatal Diagnosis of Fetal Trisomy 18 and Trisomy 13 by Maternal Plasma DNA Sequencing", PLOS ONE, vol. 6, no. 7, 6 July 2011 (2011-07-06), page e21791, XP055024137, DOI: 10.1371/journal.pone.0021791
- MATHIAS EHRICH ET AL: "Noninvasive detection of fetal trisomy 21 by sequencing of DNA in maternal blood: a study in a clinical setting", AMERICAN JOURNAL OF OBSTETRICS & GYNECOLOGY, MOSBY, ST LOUIS, MO, US, vol. 204, no. 3, 28 December 2010 (2010-12-28), pages 205.e1-205.e11, XP028184664, ISSN: 0002-9378, DOI: 10.1016/J.AJOG.2010.12.060 [retrieved on 2011-01-07]
- FAN H C ET AL: "Noninvasive diagnosis of fetal aneuploidy by shotgun sequencing DNA from maternal blood", PROCEEDINGS OF THE NATIONAL ACADEMY OF SCIENCES, NATIONAL ACADEMY OF SCIENCES, US, vol. 105, no. 42, 21 October 2008 (2008-10-21), pages 16266-16271, XP002613056, ISSN: 0027-8424, DOI: 10.1073/PNAS.0808319105 [retrieved on 2008-10-06]

EP 2 728 014 B1

- **XUE X ET AL: "Optimizing the yield and utility of circulating cell-free DNA from plasma and serum", CLINICA CHIMICA ACTA, ELSEVIER BV, AMSTERDAM, NL, vol. 404, no. 2, 27 June 2009 (2009-06-27) , pages 100-104, XP026138962, ISSN: 0009-8981, DOI: 10.1016/J.CCA. 2009.02.018 [retrieved on 2009-03-10]**

**Description**

The detection of fetal chromosomal aneuploidies is an important procedure in prenatal diagnosis. Several major diseases are caused by chromosomal aneuploidies, such as Down syndrome (also referred to as trisomy 21), trisomy 18, trisomy 13, and it is of utmost importance to predict as soon as possible whether a fetus will be affected by one of these anomalies. Moreover, the risk that a fetus will be afflicted by an aneuploidy generally increases with the mother's age. Therefore, the increase in the average age of pregnant women in most developed countries further raises the need for powerful and safe diagnostic methods for detecting fetal chromosomal aneuploidies.

The detection of fetal chromosomal aneuploidies is commonly performed through invasive procedures such as chorionic villus sampling, amniocentesis or cord blood sampling. These methods have in common that they rely on the collection of a fetal biological material (amniotic fluid, chorionic villi, cord blood) in order to obtain fetal cells, necessary for a karyotype analysis. These methods have been routinely practised for a long time. However, due to their invasiveness, they are not free of risk for the fetus and for the mother. The most frequent risk is the chance of miscarriage, close to 1% in the case of amniocentesis. Other risks are associated with these invasive procedures, such as risks of infection, transmission of a disease from the mother to the fetus (for example AIDS or hepatitis B), amniotic fluid leakage, or premature birth.

Non-invasive methods based on ultrasound scanning or on the detection of maternal serum biochemical markers have also been developed, but these methods are mainly restricted to the detection of epiphenomena, and have a limited clinical usefulness for detecting the core pathologies of chromosomal abnormalities.

The discovery of cell-free fetal nucleic acids in maternal plasma in 1997 opened up new possibilities. The first strategies using these nucleic acids for assessing the fetal chromosomal dosage were based on the analysis of the allelic ratio of SNPs in target nucleic acids (placental mRNA and DNA molecules bearing a placental-specific DNA methylation signature) based on the assessment of the fetal chromosomal dosage by allelic ratio analysis of SNPs. Another strategy was developed more recently using digital PCR (Lo et al., 2007). The technique consists in measuring the total amount of a specific locus on a potentially aneuploid chromosome (for example chromosome 21) in maternal plasma and comparing this amount to that on a reference chromosome.

In 2008, Chiu *et al* successfully implemented massively parallel sequencing in a method for diagnosing fetal trisomy 21 in maternal plasma (Chiu et al., 2008). Their method consists in performing a massively parallel sequencing on DNA extracted from the plasma samples. The sequences obtained from the MPGS step are then aligned to a reference sequence of the human genome, and the number of sequences which have been uniquely mapped to a location on the human genome, without mismatch, is counted for each chromosome, and compared to the total number of sequences obtained during the MPGS. This ratio provides an indication of the "chromosomal representation" of the DNA molecules found in a maternal plasma sample. The overrepresentation of chromosome 21 in a given sample, by comparison to a set of reference samples already known as euploid, is indicative of a fetal trisomy 21.

Approximately at the same time, Fan *et al* successfully developed another method for the diagnosis of fetal trisomy 21, using shotgun sequencing of cell-free plasma (Fan et al., 2008). After massively sequencing the cell-free DNA extracted from maternal plasma samples, Fan et al. mapped each sequence to the human genome. Each chromosome of the human genome was then divided into 50 kb bins, and, for each bin the number of sequence tags uniquely mapped to the human genome with at most one mismatch was counted. Fan et al. then calculated the median value of this count of sequence tag over each chromosome. Finally, Fan et al. compared the chromosome 21 sequence tag density of plasma issued from mothers carrying a fetus afflicted by trisomy 21 to that of plasma issued from mothers carrying euploid fetuses, and they noticed that the trisomy 21 sequence tag density was higher than that of euploid samples, with a 99% confidence level.

These techniques both rely on the detection of the overrepresentation of a given chromosome in comparison to euploid reference samples. They have provided a useful "proof-of-concept" and have paved the way for an efficient use of next-generation sequencing technology in the diagnosis of fetal aneuploidy. However, the implementation of the method in a routine clinical context requires a higher level of sensitivity and specificity than that currently described in the prior art. Other examples of tests for the diagnosis of aneuploidy based on massively parallel sequencing of cell-free DNA are described in the US patent application US 2012/270739, the International patent application WO 2011/090556, and the scientific articles Chiu *et al*., 2011, Chen *et al.,* 2011 and Ehrich *et al*., 2010.

The sensitivity of non-invasive prenatal diagnosis to detect fetal aneuploidy with whole genome next generation sequencing (WG-NGS) depends on the fetal DNA fraction in the maternal plasma, and on the sequencing depth. While the fetal DNA fraction depends on a series of largely inherent biological variables, the technical variables subject to experimental modification include i), the efficiency of the DNA extraction procedure, ii), the accuracy and throughput of NGS, namely the fraction of sequence tags with unique exact matches that can be aligned to the sequenced genome (termed "unique exact sequences without mismatches" or "UES") and the total number of molecules sequenced iii), the nature of the bioinformatic algorithms, and iv), the control group of samples from pregnant women with normal fetal caryotypes that provides the reference set. The latter is of utmost importance, since individual molecules counting for each single chromosome is normalized with the median sequence tag density of all autosomes (Fan *et al* 2008). DNA extraction procedures are, for instance, discussed in Xue *et al.*, 2009.

The present invention implements a DNA extraction method not previously used for this application and having a fivefold greater yield than standard methods, together with a rigorously quality-controlled WG-NGS work-flow with overall 25-30% more UESs than the published references, and average total count of UESs of more than $15 \cdot 10^6$, which is three times higher than the current standard. The final readout of the test fits the requirements of a robust clinical test, i.e. a 100% sensitivity and 100% specificity for the major fetal aneuploidies. This procedure for instance discriminates trisomy 21 or Down syndrome from normal male and female caryotypes with $\leq 1.1 \cdot 10^{-5}$ prior probability of generating false results by chance. Since the benchmark is $\leq 2.7 \cdot 10^{-3}$, it represents an improvement of two orders of magnitude. This invention provides a combination of methods that allow the constitution of a high quality reference set of sequences, which is the key step towards defining the performance of the WG-NGS procedure.

A first aspect disclosed herein thus relates to a method for obtaining a set of reference samples and/or a set of reference parameters for the diagnosis of fetal aneuploidy from a maternal biological sample, preferably a blood sample, comprising:

- a step of extracting cell-free DNA from a set of biological samples, preferably blood samples, obtained from euploid pregnant women carrying a euploid fetus;

- a step of performing a massively parallel sequencing of DNA of each sample;

- a step of mapping the obtained sequences to the human genome for each sample;

- optionally calculating a set of reference parameters, wherein each reference parameter is indicative of the number of unique exact sequences mapped to a chromosome or chromosomal region of interest for each sample;

- obtaining a set of reference samples and/or a set of reference parameters;

wherein the method comprises at least one of the following additional steps/features:

- the extraction of cell-free DNA from each biological sample comprises:

  ◦ mixing said biological sample with a composition comprising chloroform and phenol;

  ◦ extracting the aqueous phase from said mixture;

  ◦ precipitating DNA from said aqueous phase;

  ◦ optionally collecting precipitated DNA.

- After the extraction step, analyzing the size distribution of the DNA molecules within each sample and selecting a set of samples based on the size distribution of the DNA molecules within said samples;

- After the extraction step or after the selection step based on the size distribution of the DNA molecules, pre-sequencing DNA of each sample, mapping the obtained sequences to the human genome, and selecting a set of samples based on the amount of unique exact sequences mapped to the human genome;

- After the step of mapping the sequences obtained from massively parallel sequencing, selecting a set of samples based on the number of unique exact sequences mapped to the human genome.

The method can comprise any one of these additional steps or features, any combination of two or three of these additional steps or features or the four additional steps and features.

The present invention relates to a method for obtaining a set of reference samples for the diagnosis of fetal aneuploidy from a maternal biological sample containing cell-free DNA, comprising:

(i) extracting cell-free DNA from a set of biological samples, preferably blood samples, obtained from a set of euploid pregnant women carrying a euploid fetus;

(ii) pre-sequencing DNA of each sample;

(iii) mapping the sequences obtained in step (ii) to the human genome;

(iv) selecting a set of samples based on the amount of unique exact sequences mapped to the human genome in step (iii);

(v) massively parallel sequencing DNA of each sample from said set of samples selected in step (iv);

(vi) mapping the sequences obtained in step (v) to the human genome;

(vii) selecting a set of reference samples based on the number of unique exact sequences mapped to the human genome in step (vi).

In a specific embodiment, the method comprises, between the step of extracting cell-free DNA and the step of pre-sequencing:

-    analyzing the size distribution of the DNA molecules within each sample;

-    selecting a set of samples based on the size distribution of the DNA molecules within said samples.

In a specific embodiment, the step of selecting a set of samples based on the size distribution of the DNA molecules comprises selecting samples in which at least 90 wt%, preferably more than 95wt% of the DNA molecules have a size from 156 bp to 176 bp.
In another embodiment, the step of selecting a set of samples based on the size distribution of the DNA molecules comprises selecting samples with at least 0.88 ng/$\mu$l DNA molecules with a size from 156 bp to 176 bp.
In another embodiment, step (ii) comprises sequencing from 1000 to 100000 sequences within each sample.
In another embodiment, step (iv) comprises selecting samples having at least 70 % of unique exact sequences with respect to the total number of sequences obtained in step (ii).
In another embodiment, step (v) comprises sequencing at least 25 million sequences for each sample.
In another embodiment, step (v) comprises obtaining at least 25 million filter passing reads for each sample.
In another embodiment, step (vii) comprises selecting samples having more than 15 millions unique exact sequence reads.
The present invention also refers to a method for diagnosing fetal aneuploidy from a maternal biological test sample, preferably a blood sample, comprising:

(a) extracting cell-free DNA from a maternal biological test sample obtained from a pregnant woman;

(b) massively parallel sequencing cell-free DNA extracted from said test sample;

(c) mapping the sequences obtained in step (b) to the human genome;

(d) calculating a test parameter indicative of the number of unique exact sequences mapped to a chromosome or chromosomal region of interest;

(e) calculating a set of reference parameters, wherein each reference parameter is indicative of the number of unique exact sequences mapped to a chromosome or chromosomal region of interest for a sample of a set of reference samples, such as a set of euploid reference samples, for example as obtained according to the present invention;

(f) Comparing said test parameter calculated in step (d) with said set of reference parameters calculated at step (e);

(g) based on the comparison, diagnosing a fetal aneuploidy.

Preferably, the extraction of cell-free DNA from the maternal biological test sample comprises:

-    mixing said biological sample with a composition comprising chloroform and phenol;

-    extracting the aqueous phase from said mixture;

-    precipitating DNA from said aqueous phase;

- optionally collecting precipitated DNA.

In a specific embodiment, said test parameter is the unique sequence tag density of the chromosome or chromosomal region of interest normalized to the median unique exact sequence tag density of all autosomes.

In another embodiment, said test parameter is the percentage of unique exact sequences mapped to said chromosome or chromosomal region, with respect to the total number of unique exact sequences mapped to all chromosomes, or to the total number of unique exact sequences mapped to all autosomes.

In another embodiment, the comparison in step (f) is made through calculation of the z-score of said test parameter with respect to the set of reference parameters.

In another embodiment, the chromosome of interest is chromosome 21, chromosome 18 or chromosome 13.

In another embodiment, the chromosome of interest is chromosome 21, and the z-score of a trisomy 21 sample is at least 4.4 while the absolute value of the z-score of a sample euploid for chromosome 21 is less than 4.4.

Also disclosed is a method for extracting cell-free DNA from a maternal biological sample containing fetal and maternal cell-free DNA, comprising:

- mixing said biological sample with a composition comprising chloroform and phenol;
- extracting the aqueous phase from said mixture;
- precipitating DNA from said aqueous phase;
- optionally collecting precipitated DNA.

Also disclosed is the use of chloroform and phenol, preferably of a composition comprising chloroform and phenol for extracting cell-free DNA from a maternal biological sample containing fetal and maternal cell-free DNA.
In a specific aspect, said use is in a method for obtaining a set of reference samples for the diagnosis of fetal aneuploidy from a maternal biological sample.
In another aspect, said use is in a method for diagnosing fetal aneuploidy from a maternal biological test sample

Also disclosed is a set of reference samples obtainable according to the method of the present invention.

Also disclosed is a computer program product for implementing one or more steps of the method for obtaining a set of reference samples for the diagnosis of fetal aneuploidy from a maternal biological sample.

Also disclosed is a computer program product for implementing one or more steps of the method for diagnosing fetal aneuploidy from a maternal biological test sample, for example one or more of step (d) to (g).

Also disclosed is a kit comprising one or more of:

- one or more compositions and/or a kit for extracting cell-free DNA, for example including a composition comprising phenol and chloroform;

- a set of reference samples obtainable according to the method of the present invention;

- a set of reference parameters obtainable according to the method according to the present invention, optionally included in a physical support, such as a computer readable media;

- a computer program product for implementing one or more steps of the method for obtaining a set of reference samples for the diagnosis of fetal aneuploidy from a maternal biological sample;

- a computer program product for implementing one or more steps of the method for diagnosing fetal aneuploidy from a maternal biological test sample.

## Brief Description of the drawings

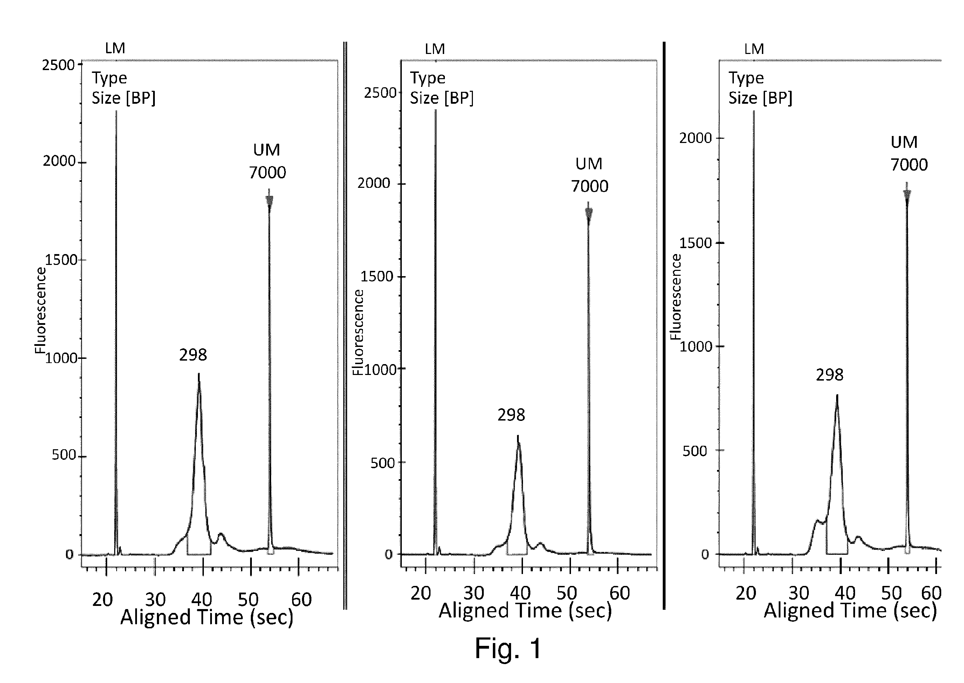

**Figure 1:** size distribution of 3 maternal plasma samples as obtained by capillary electrophoresis. The DNA molecules in these samples are ligated to a 132 bp sequencing adaptor/barcode.

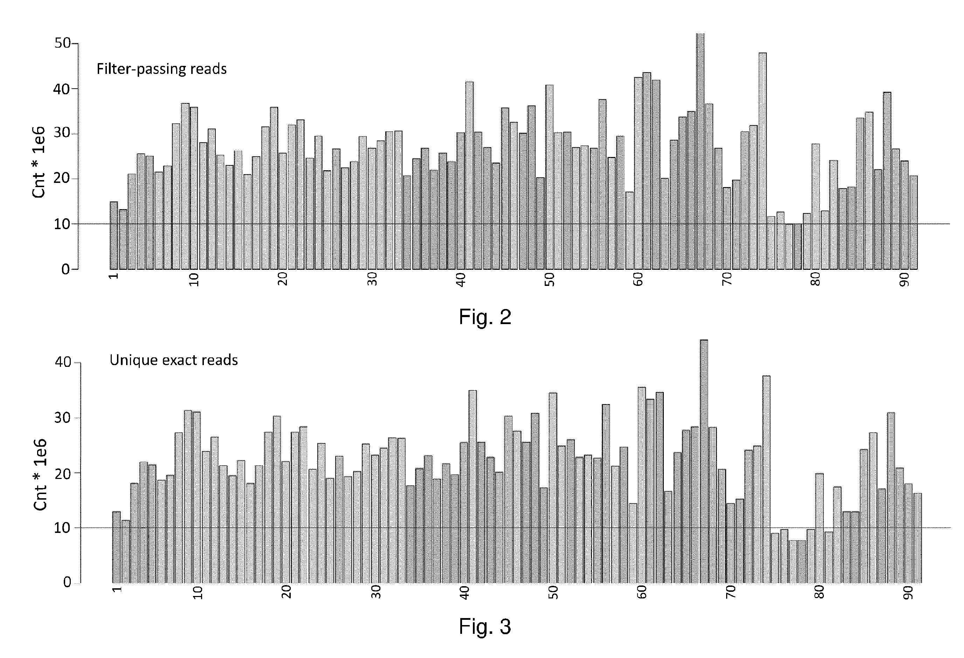

**Figure 2:** total number of filter passing sequence reads obtained by WG-NGS sequencing for 91 samples (euploid and aneuploid). The axis legend in ordinate reads "Cnt +1e6", namely the sequence count in million.

**Figure 3:** number of unique exact sequences for the same samples shown in Fig. 2. The axis legend in ordinate reads "Cnt +1 e6", namely the sequence count in million.

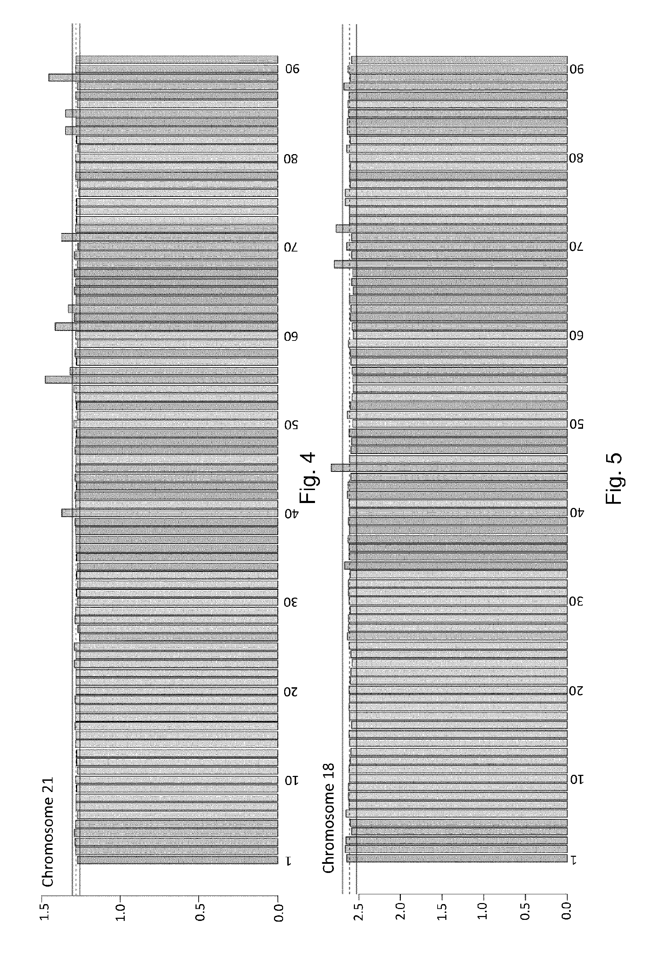

**Figure 4:** percentage of total unique sequence reads mapped to chromosome 21 with 1/100,000 confidence interval (z-score=4.4) with respect to known healthy individuals (reference samples selected according to the method of the present invention). The horizontal middle dotted line corresponds to the mean percentage of the reference sample. The horizontal full lines above and below the dotted line correspond to the discrimination threshold (mean $\pm$ 4.4* SD). The trisomy 21 samples are positively discriminated.

**Figure 5:** percentage of total unique sequence reads mapped to chromosome 18 with 1/100,000 confidence interval (z-score=4.4) with respect to known healthy individuals (reference samples selected according to the method of the present invention). The horizontal middle dotted line corresponds to the mean percentage of the reference sample. The horizontal full lines above and below the dotted line correspond to the discrimination threshold (mean $\pm$ 4.4* SD). The trisomy 18 samples are posititively discriminated.

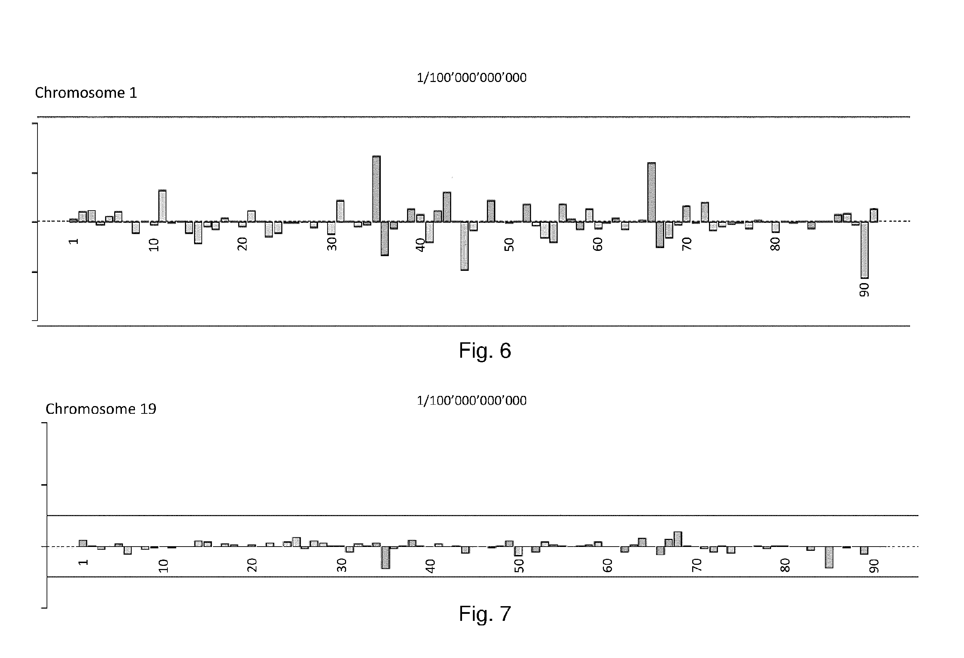

**Figure 6:** Scores of chromosome 1 using a second scoring algorithm. The discrimination thresholds correspond to a 1/100,000,000,000 confidence interval with respect to known healthy individuals (reference samples selected according to the method of the present invention).

**Figure 7:** Scores of chromosome 19 score using a second scoring algorithm. The discrimination thresholds correspond to a 1/100,000,000,000 confidence interval with respect to known healthy individuals (reference samples selected according to the method of the present invention).

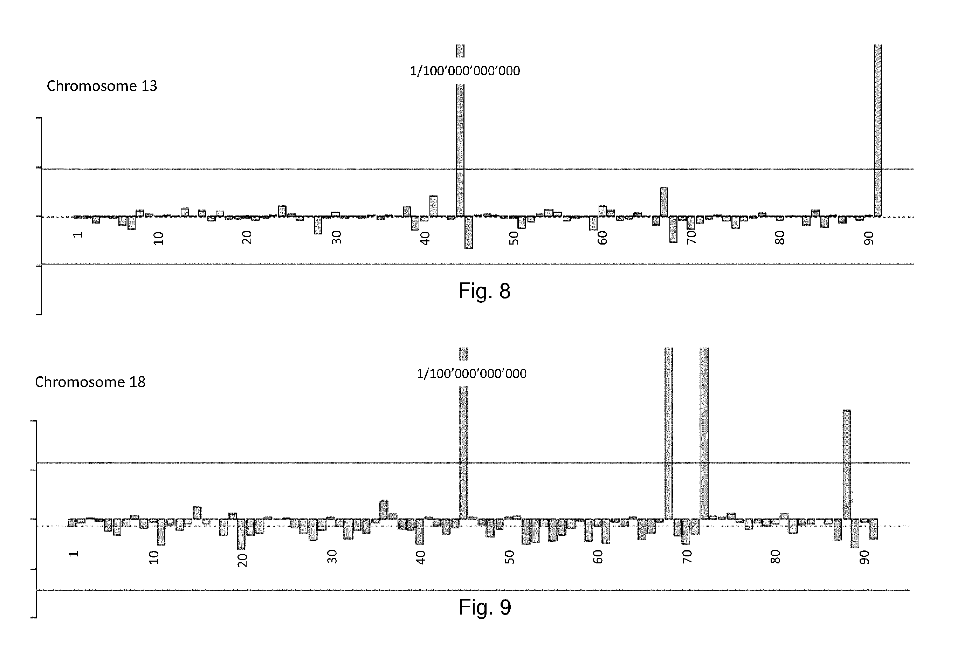

**Figure 8:** Scores of chromosome 13 score using a second scoring algorithm. The discrimination thresholds correspond to a 1/100,000,000,000 confidence interval with respect to known healthy individuals (reference samples selected according to the method of the present invention). The trisomy 13 sample is positively discriminated.

**Figure 9:** Scores of chromosome 18 using a second scoring algorithm. The discrimination thresholds correspond to a 1/100,000,000,000 confidence interval with respect to known healthy individuals (reference samples selected according to the method of the present invention). The trisomy 18 samples are positively discriminated.

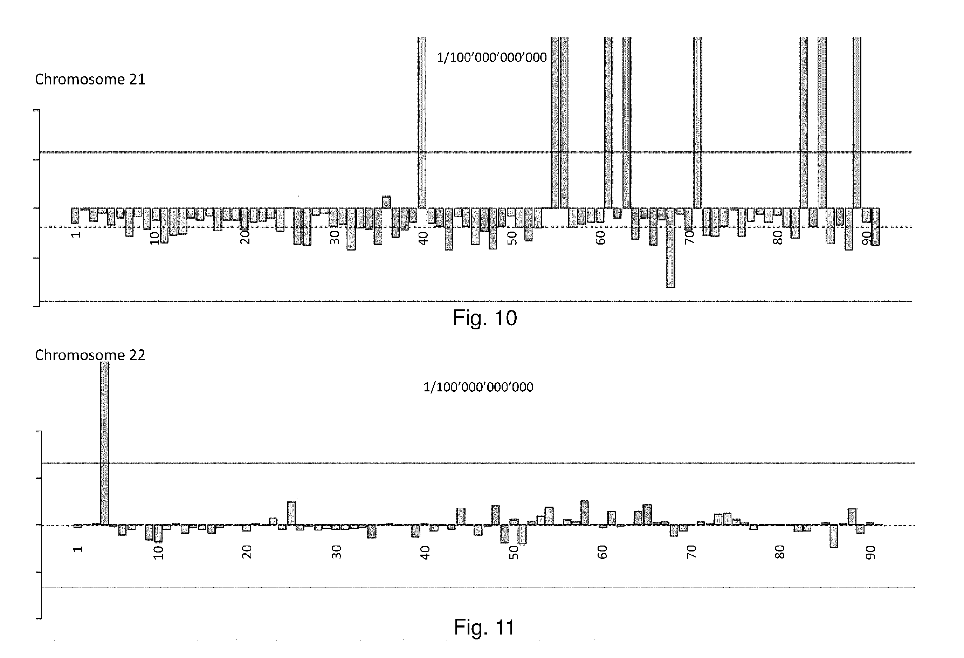

**Figure 10:** Scores of chromosome 21 using a second scoring algorithm. The discrimination thresholds correspond to a 1/100,000,000,000 confidence interval with respect to known healthy individuals (reference samples selected according to the method of the present invention). The trisomy 21 samples are positively discriminated.

**Figure 11:** Scores of chromosome 22 using a second scoring algorithm. The discrimination thresholds correspond to a 1/100,000,000,000 confidence interval with respect to known healthy individuals (reference samples selected according to the method of the present invention). The trisomy 22 sample is positively discriminated.

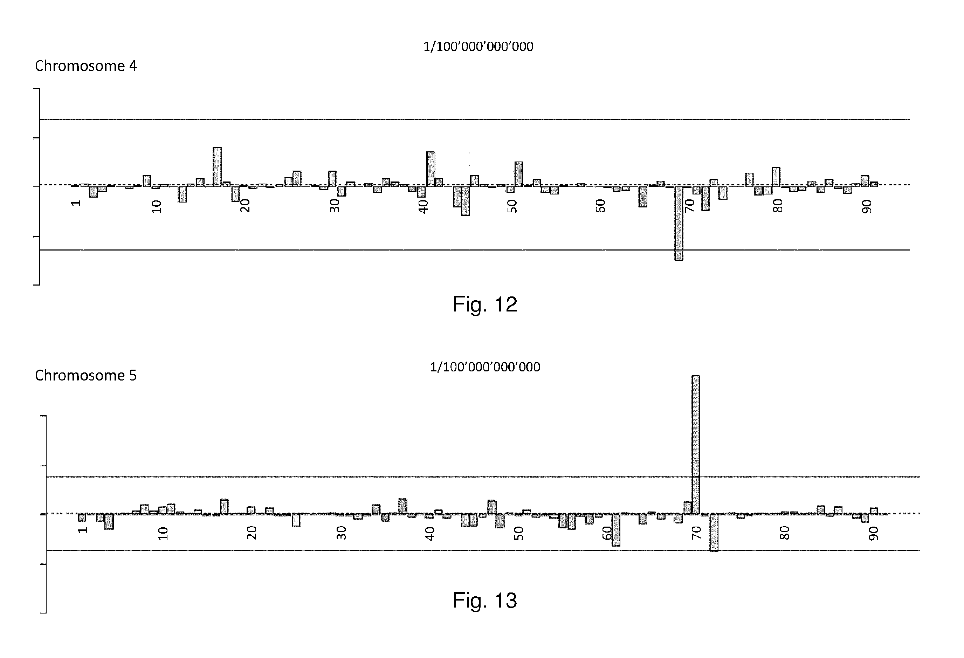

**Figure 12:** Scores of chromosome 4 using a second scoring algorithm. The discrimination thresholds correspond to a 1/100,000,000,000 confidence interval with respect to known healthy individuals (reference samples selected according to the method of the present invention). The 4p microdeletion (Wolf-Hirschhorn syndrome) sample is negatively discriminated.

**Figure 13:** Scores of chromosome 5 using a second scoring algorithm. The discrimination thresholds correspond to a 1/100,000,000,000 confidence interval with respect to known healthy individuals (reference samples selected according to the method of the present invention). The 5p microdeletion/duplication (cri du chat syndrome) sample is positively discriminated.

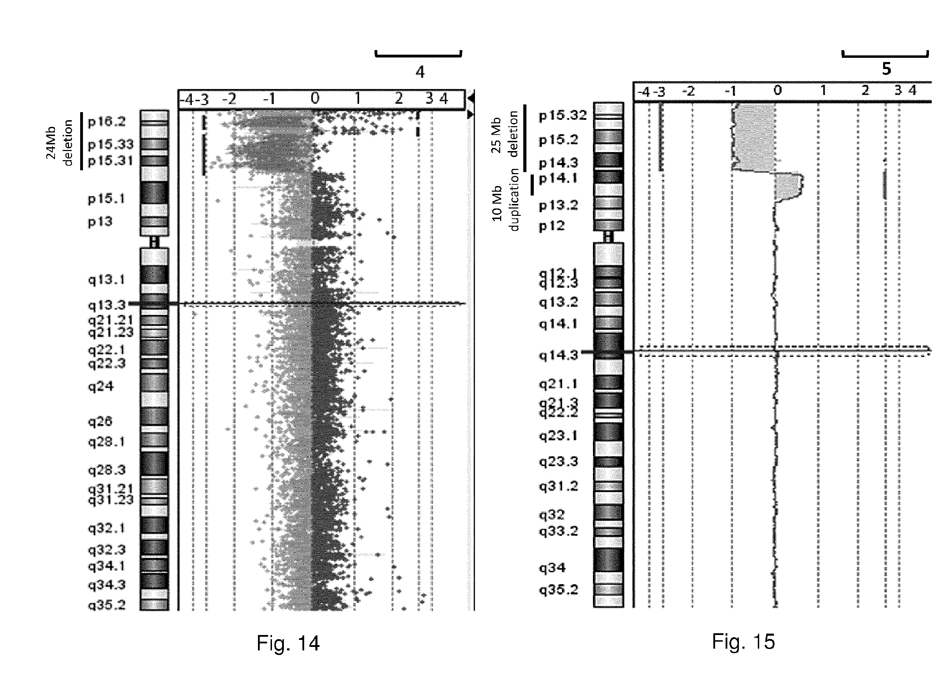

**Figure 14:** Sequence tag densities over chromosome 4 of a 4p deletion syndrome sample. A negative deviation from the mean density of the reference samples is apparent at the location of the 4p deletion.

**Figure 15:** Sequence tag densities over chromosome 5 of a 5p deletion syndrome sample. Positive and negative deviations from the mean density of the reference samples are apparent at the location of the 5p microdeletion and duplication, respectively.

The data shown on Figures 2 to 13 were all obtained with the same set of 91 samples, and are shown in the same order on each Figure. The ID of every 10 samples is indicated below the bars. The karyotype of specific samples (samples 2, 3, 4, 26, 40, 44, 45, 55, 56, 61, 63, 68, 69, 70, 71, 83, 85, 88, 89, 90, 91) is indicated inside or above the corresponding bar. These karyotypes are also listed in Table 5 (text identical to that of the Figures).

## Definitions

As used herein the terms "**next-generation sequencing**" (NGS), "**whole-genome next-generation sequencing**" (WG-NGS) or "**massively parallel sequencing**" are synonyms and refer to a high-throughput sequencing method in which hundreds of thousands of sequencing processes are made parallel. Next-generation sequencing methods are useful for obtaining several millions of sequences in a single run. These methods include: Single-molecule real-time sequencing, Ion semiconductor sequencing, pyrosequencing, sequencing by synthesis, sequencing by ligation.

As used herein the term "**Cell-free DNA**" refers to a DNA molecule or a set of DNA molecules freely circulating in a biological sample, for example in blood. A synonym is "circulating DNA". Cell-free DNA is extracellular, and this term is used as opposed to the intracellular DNA which can be found, for example, in the cell nucleus or mitochondria.

As used herein the term **aneuploidy** refers to the variation of a quantitative amount of one chromosome from that of a diploid genome. The variation may be a gain, or a loss. It may involve a whole chromosome or a part thereof, for example only a chromosomal region. Aneuploidy can include monosomy (lack of one chromosome), partial monosomy (translocation or deletion of a portion of a chromosome), trisomy (gain of one extra chromosome), partial trisomy (gain and/or duplication of a portion of a chromosome).

**Euploidy** is herein used to mean the contrary of aneuploidy, i.e. a euploid sample refers to a diploid genome, chromosome or chromosomal portion. For instance, an individual euploid for chromosome 21 has two copies of the chromosome 21.

Examples of monosomy or partial monosomy include Wolf-Hirschhorn syndrome, cri du chat syndrome, 5q deletion syndrome, Williams syndrome, Jacobsen syndrome, Angelman syndrome, Prader-Willi syndrome, Miller-Dieker syndrome, Smith-Magenis syndrome, 18q deletion syndrome, DiGeorge syndrome.

Examples of trisomy include trisomy 1, trisomy 2, trisomy 3, trisomy 4, trisomy 5, trisomy 6, trisomy 7, trisomy 8 (Warkany syndrome), trisomy 9, trisomy 10, trisomy 11, trisomy 12, trisomy 13 (Patau syndrome), trisomy 14, trisomy 15, trisomy 16, trisomy 17, trisomy 18 (Edwards syndrome), trisomy 19, trisomy 20, trisomy 21 (Down syndrome), trisomy 22.

Other examples of disorders involving a loss (deletion) of one or several chromosomal regions include 1p36 deletion syndrome, TAR deletion, 1q21.1 deletion, 2q11.2 deletion, 2q11.2q13 deletion, 2q13 deletion, 2q37 deletion, 3q29 deletion, Wolf-Hirschhorn deletion, Sotos syndrome deletion, 6q16 deletion, Williams syndrome deletion , WBS-distal deletion, 8p23.1 deletion, 9q34 deletion, 10q23 deletion, Potocki-Shaffer syndrome, SHANK2 FGFs deletion, 12q14 deletion syndrome, 13q12 deletion, 15q11.2 deletion, Prader-Willi/Angelman syndrome, 15q13.3 deletion, 15q24 BP0-BP1 deletion, 15q24 BP0-BP1 deletion, 15q24 BP2-BP3 deletion, 15q25.2 deletion, Rubinstein-Taybi syndrome, 16p13.11 deletion, 16p11.2p12.1 deletion, 16p12.1 deletion, 16p11.2 distal deletion, 16p11.2 deletion, 17p13.3 deletion, 17p13.3 deletion, HNPP, Smith-Magenis syndrome deletion, NF1 deletion syndrome, RCAD (renal cysts and diabetes), 17q21.31 deletion, DiGeorge/VCFS deletion, 22q11.2 distal deletion, Phelan-McDermid syndrome.

Other examples of disorders involving a gain (duplication) of one or several chromosomal regions include 1p36 duplication, 1q21.1 duplication, 2q11.2 duplication, 2q11.2q13 duplication, 2q13 duplication, 2q37 duplication, 3q29 duplication, Wolf-Hirschhorn region duplication, 5q35 duplication, 6q16 duplication, Williams syndrome duplication, WBS-distal duplication, 8p23.1 duplication, 9q34 duplication, 10q23 duplication, 11p11.2 duplication, SHANK2 FGFs duplication, 12q14 duplication, 13q12 duplication, 15q11.2 duplication, Prader-Willi/Angelman region duplication, 15q13.3 duplication, 15q24 BP0-BP1 duplication, 15q24 BP2-BP3 duplication, 15q25.2 duplication, Rubinstein-Taybi region duplication, 16p13.11 duplication, 16p11.2p12.1 duplication, 16p12.1 duplication, 16p11.2 distal duplication, 16p11.2 duplication, 17p13.3 duplication, 17p13.3 duplication, 17p13.3 duplication, CMT1A, Potocki-Lupski syndrome, NF1 duplication, 17q12 duplication, 17q21.31 duplication, 22q11.2 duplication, 22q11.2 distal duplication, 22q13 duplication.

Reference on these disorders along with a comprehensive review of aneuploidy-related genomic disorders involving a copy number variation of chromosomal portions of less than 10 Mb, can be found in Cooper et al., 2011.

As used herein, the term "**euploid sample**" refers to a sample obtained from a euploid mother carrying a euploid fetus. The term "euploid" can be used with a relative sense, i.e. relating to a specific chromosome or chromosomal region of interest. Alternatively, the term "euploid" can be used with an absolute sense, i.e. relating to the whole genome. In this case, a euploid sample is not afflicted by any aneuploidy over its whole genome.

As used herein, the term "**aneuploid sample**" refers to a sample obtained from a euploid mother carrying an aneuploid fetus. Similarly to "euploid', the term "aneuploid" can be used with reference to a specific chromosome or chromosomal region of interest, or with reference to the whole genome.

As used herein, the term "**unique exact sequence**" refers to a sequence uniquely mapped to the human genome without any mismatch. In other words, the sequence has been aligned with a single location in the human genome, and has exactly the same sequence as said location, i.e. without any deletion, addition or mutation with respect to the sequence found at said location in the human genome. The unique exact sequence generally has a length of 20 to 100 bp, preferably 40 to 70 bp, still preferably 50 bp.

As used herein, a "**maternal sample**" such as in "maternal biological sample" is a sample obtained from a pregnant woman.

As used herein, a "**biological sample**" preferably refers to a biological sample containing cell-free DNA, still preferably refers to a whole blood, plasma, serum, urine or breast milk sample.

## Detailed description of the invention

A first aspect of the invention refers to the constitution of a set of euploid reference biological samples, wherein each reference sample is carefully chosen so as to increase the statistical confidence of a fetal aneuploidy diagnosis method. The workflow of this selection process comprises several important selection steps:

-    a selection based on the size distribution of DNA inside the samples;

-    a selection based on the quantity of unique exact sequences, obtained by pre-sequencing the samples, and mapping the obtained sequences on the human genome (steps (ii) to (iv));

-    a selection based on the quantity of unique exact sequences, obtained by performing the sequencing of the samples, and mapping the obtained sequences on the human genome (steps (v) to (vii));

The method according to the present invention can comprise any of the three above-mentioned selection steps. However, in a preferred embodiment, all three selection steps are performed, thus increasing the quality of the final set of reference samples.

Biological sample collection

The methods according to the present invention can generally be performed on any biological sample in which cell-free DNA, in particular fetal and maternal cell-free DNA can be found. The biological sample can especially be a bodily fluid such as blood, urine, breast milk. A blood sample is preferred. As referred herein, a blood sample refers to a whole-blood sample, a plasma sample or a serum sample. The biological samples can be collected at any time during the pregnancy, but are preferably collected from 7 weeks of pregnancy, for example between 7 weeks and 20 weeks of pregnancy, preferably from 7 to 14 weeks of pregnancy, still preferably from 7 to 10 weeks of pregnancy. A diagnosis performed as early as 7 weeks of pregnancy provides the advantage of keeping more medical options opened in cases where a decision to interrupt the pregnancy is taken (for example, an interruption through the use of a drug or a combination of drugs may be allowed depending on the national laws).

The biological samples can be collected following an invasive prenatal procedure, such as chorionic villus sampling, amniocentesis, or cord blood sampling. They can be collected at any time following the invasive procedure, for example at least 10 min, 20 minutes or 30 minutes following the invasive procedure. The biological samples can also be collected at least one or more days following the invasive procedure, for example from two to five days following the invasive procedure.

Alternatively, the biological samples can be collected from women not yet having experienced an invasive prenatal procedure. This situation is preferable for the biological samples to be diagnosed, as an advantage of the method is precisely to avoid any invasive procedure.

The aneuploidy status of the fetus in samples intended to form the reference set can be diagnosed independently from the method according to the present invention. This may be useful for ascertaining that the samples used for forming the reference set of samples are indeed euploid samples, or in other words, samples obtained from euploid mothers carrying a euploid fetus. The euploid samples used for obtaining the reference set of samples are preferably euploid with reference to the "absolute" definition of the term, as given above, i.e. they are euploid over the whole genome, and not only for a specific chromosome of interest.

A method for assessing the aneuploidy status of the fetus can comprise collecting fetal cell material from the mother by an invasive prenatal diagnosis procedure, such as amniocentesis, chorionic villus sampling or cord blood sampling. The aneuploidy status of the fetus can then be assessed by any of following techniques: karyotyping, Fluorescence In Situ Hybridization (FISH), Quantitative Polymerase Chain Reaction (PCR) of Short Tandem Repeats, Quantitative Fluorescence PCR (QF-PCR), Quantitative Real-time PCR (RT-PCR) dosage analysis, Quantitative Mass Spectrometry of Single Nucleotide Polymorphisms, and Comparative Genomic Hybridization (CGH).

In most cases, the aneuploidy status of the mother is already known, because most aneuploidy-related diseases are symptomatic. However, if needed, the aneuploidy status of the mother can also be assessed by using cell material obtained from the mother. Any of the aforementioned techniques can be employed.

Cell-free DNA Extraction

An important parameter of the method according to the invention is an efficient DNA extraction from the maternal biological samples. Cell-free DNA extraction is preferably performed via a protocol of phenol-chloroform extraction. The extraction protocol typically comprises:

- mixing said biological sample with a composition comprising chloroform and phenol;

- extracting the aqueous phase from said mixture;

- precipitating cell-free DNA from said aqueous phase;

- optionally collecting cell-free DNA.

The present invention encompasses the use of phenol/chloroform for extracting cell-free DNA from a biological sample, preferably from a blood sample such as a plasma sample. The method is particularly appreciable for extracting mixed fetal and maternal cell-free DNA from a maternal biological sample, as it yields a more robust fetal DNA signal than the existing methods. According to the present invention, the term "phenol/chloroform" refers to a mixture of phenol and chloroform, i.e. to a composition comprising phenol and chloroform. Said composition is preferably an aqueous solution and preferably also comprises isoamyl alcohol. The pH of the composition is preferably from 7 to 9, still preferably from 7.8 to 8.2. A preferred composition is a 25:24:1 mixture of phenol:chloroform:isoamyl alcohol at a pH from 7.8 to 8.2. The composition may comprise one or more additives, such as one or more antioxidants and/or stabilizers.
In a specific embodiment, the extraction method comprises a step of pre-treating the biological sample with one or more proteases, such as proteinase K.
The extraction of the aqueous phase may comprise centrifuging the biological sample mixed with chloroform and phenol, and collecting the aqueous phase. The centrifugation provides a separation of the mixed biological sample into a lower organic phase, comprising mainly phenol, proteins or protein debris, and an upper aqueous phase comprising nucleic acids.
In an embodiment, the precipitation of cell-free DNA from the aqueous phase comprises the steps of:

- mixing at least one precipitation agent with the aqueous phase;

- centrifuging said mixed aqueous phase; and

- collecting the centrifugation pellet.

The precipitation agent is preferably selected from glycogen, a lower alcohol such as isopropanol or ethanol, or mixtures thereof. The centrifugation pellet containing DNA can then be washed one or more time, for example with ethanol and/or ether. Finally, DNA can be resuspended in a suspension buffer, for example a Tris buffer.
The phenol-choloroform extraction protocol yields a fivefold higher amount of DNA than the column methods classically employed in the context of fetal aneuploidy detection using massively parallel sequencing (Chiu et al., 2008, Fan et al., 2008). It also yields a higher fraction of DNA at a size of 156-176 bp, i.e. maternal and fetal cell-free DNA. This protocol is thus an important tool for increasing the number of sequence reads originating from fetal DNA.

Preparation of the sequencing library

Following cell-free DNA extraction, the samples containing extracted DNA are optionally processed for preparing the sequencing library. The preparation can include one or more amplification steps, a ligation with one or more sequencing adaptors, and/or barcoding the DNA molecules. A typical workflow of the sequencing library preparation includes a step of ligation of one or more adaptor sequences, optionally linked to one or more barcode sequences, to the DNA molecules inside the sample, followed by an amplification of the adaptor/barcode-ligated DNA molecules.
Sequencing adaptors are short nucleotide sequences which are commonly used in modern sequencing technologies. The adaptors are used for anchoring the DNA molecules to be sequenced to a solid surface, for example in a flow cell. These adaptors are thus designed so as to hybridize to target oligonucleotides tethered to the solid surface. The ligation of adaptors is preferably performed by repairing the ends of the DNA molecules, i.e. suppressing or filling out the overhangs of the extracted DNA molecules, for example through the action of one or more exonucleases and/or polymerases, thus yielding blunt-ended DNA molecules. An overhang of 'A' bases is then added at the 3' end of the blunt-ended DNA molecules. The adaptors containing an overhang of 'T' bases at their 3' end, are then added and are ligated to the overhang of 'A' bases at the 3'end of the DNA molecules.

The DNA fragments within the sample can also be barcoded. Barcoding refers to the ligation of a sample-specific tag to the DNA molecules of a sample. Barcoding allows the sequencing of several samples in a single sequencing run, which saves time and resources.

The DNA fragments inside the sample can also be subjected to one or more amplification cycles, for example by PCR. From 10 to 25 amplification cycles, for example 18 amplification cycles may be run. The amplification is preferably carried out after the ligation of an adaptor sequence to the DNA molecules. The PCR amplification preferably uses primers against the adaptor sequence, thus enriching the library into adaptor-ligated fragments.

Cell-free DNA size distribution analysis and selection

In an embodiment, the size distribution of the DNA molecules within each sample is analyzed. This analysis is preferably performed by capillary electrophoresis. It is for example carried out by using a commercial lab-on-a-chip capillary electrophoresis system. The size distribution analysis can be conducted before the preparation of the sequencing library. However, it is preferably performed after the preparation of the sequencing library, most preferably after having amplified the DNA samples, so as to provide sufficient DNA material for a capillary electrophoresis assay.

By analyzing the size distribution of the DNA molecules in a set of euploid samples, the inventors of the present application have found that the size distribution had a size peak at about 298 bp (Figure 1). After subtraction of the adaptor/barcode sequence size of 132 bp, the peak size corresponds to 166 bp. This value is in agreement with the data previously provided by Fan et al., 2008 and also with the hypothesis of a mainly mononucleosomal origin of cell-free DNA.

According to the present invention, the size distribution of DNA within the samples is used as a criterion in the process of composing an appropriate set of reference samples for the diagnosis of fetal aneuploidy. This criterion allows the selection of samples with a high level of cell-free DNA and the elimination of the samples with a low level of cell-free DNA.

A selection criterion may consist in the occurrence of a size peak at about 166 bp. As used herein, the term "about 166 bp" can have the meaning of "from 151 to 181 bp", or "from 156 to 176 bp", or "from 161 to 171 bp" or "from 163 to 169 bp" or "from 165 to 167 bp". Alternatively, this term can have the meaning of "at exactly 166 bp".

Another criterion for selecting appropriate reference samples may consist in the height of the peak at about 166 bp, or, in other terms, in the fraction of DNA molecules having a size of about 166 bp. Accordingly, in a specific embodiment, the step of of selecting a set of samples based on the size distribution of the DNA molecules comprises selecting the samples wherein at least 80 wt%, still preferably at least 90 wt%, preferably at least 95 wt%, still preferably at least 97wt% of the DNA molecules inside the sample have a size of about 166 bp, preferably from 156 to 176 bp.

Alternatively or in addition, the step of selecting a set of samples based on the size distribution of the DNA molecules comprises selecting samples wherein the concentration of DNA molecules with a size of about 166 bp, preferably from 156 to 176 bp, is of at least 0.88 ng/$\mu$l, preferably at least 0.90 ng/$\mu$l, still preferably at least 0.95 ng/$\mu$l or at least 1.00 ng/$\mu$l or at least 1.05 ng/$\mu$l or at least 1.10 ng/$\mu$l.

Alternatively or in addition, the step of selecting a set of samples based on the size distribution of the DNA molecules comprises selecting samples wherein the quantity of DNA molecules with a size of about 166 bp, preferably from 156 to 176 bp, is of at least 13 ng, preferably at least 13.5 ng, still preferably at least 14.25 ng or at least 15 ng or at least 15.75 ng or at least 16.5 ng.

Preferably, the mean concentration of extracted DNA molecules with a size of about 166 bp, preferably from 156 to 176 bp, among the set of samples selected in the step of selecting a set of samples based on the size distribution of the DNA molecules is of at least 0.88 ng/$\mu$l, preferably at least 0.90 ng/$\mu$l, still preferably at least 0.95 ng/$\mu$l or at least 1.00 ng/$\mu$l or at least 1.05 ng/$\mu$l or at least 1.10 ng/$\mu$l.

Preferably, the mean quantity of DNA molecules with a size of about 166 bp, preferably from 156 to 176 bp, among the set of samples selected in the step of selecting a set of samples based on the size distribution of the DNA molecules is of at least 13 ng, preferably at least 13.5 ng, still preferably at least 14.25 ng or at least 15 ng or at least 15.75 ng or at least 16.5 ng.

The concentration and/or quantity is preferably measured on DNA libraries prepared for the sequencing step. The concentration and/or quantity is still preferably measured on adaptor/barcode-ligated DNA molecules, for instance on DNA molecules ligated with a 132 bp adaptor/barcode. Preferably, the DNA molecules have been submitted to 18 amplification cycles after the ligation of the adaptor/barcode. Still preferably, the concentration and/or quantity is measured on DNA libraries prepared using the Illumina's ChIP sequencing protocol by using 20 ng DNA as input material.

Interestingly, the inventors of the present application have also discovered that the DNA molecules in plasma maternal samples presents a smaller sized shoulder at about 133 to 143 bp (Figure 1, right panel). This shoulder likely reflects fetal DNA, and can be used as an additional or alternative quality control criterion for selecting samples having an enriched fetal DNA fraction. Accordingly, the step of selecting a set of samples based on the size distribution of the DNA molecules may also comprise selecting samples whose DNA size distribution reveals a peak or shoulder between 133 and 143 bp.

The size values indicated above (a peak at 166 bp, and the associated values) correspond to non-adaptor or barcode ligated DNA molecules, i.e. to the DNA molecules as found in maternal blood. If needed, these values may be adapted for taking into account the presence of an adaptor, barcode, or of any sequence tag at one or both ends of the DNA molecules.

As used herein, a peak refers to a local maximum in the curve representing the size distribution of DNA molecules inside a sample. A shoulder refers to an inflection point in this curve.

Pre-sequencing

According to the present invention, pre-sequencing refers to a small-scale sequencing which is performed prior to a larger scale next-generation sequencing. Therefore, contrary to the methods of the prior art, the method according to the present invention is characterized by two sequencing steps successively performed on each sample of the reference set. Accordingly, "pre-sequencing" can also be referred as "first sequencing". In a similar way, "massively parallel sequencing" can be referred as "second sequencing".

The inventors have postulated that the proportion of unique exact sequences within a small library of sequences would be representative of the proportion of unique exact sequences in the full scale library obtained by next-generation sequencing. Thus, by conducting a small scale sequencing of the DNA samples at an early stage, it is possible to eliminate early on, the samples having an insufficient amount of unique exact sequences. This pre-sequencing step is much less time and cost-consuming than the massively parallel sequencing which is then performed. Thus, the present invention enables time and resources to be saved while eliminating samples with an insufficient quality, thereby yielding a reference set of increased quality.

Preferably, the pre-sequencing step comprises sequencing from 1000 to 100,000 sequences per sample, still preferably from 5000 to 50000 sequences per sample.

The size of each sequence read is preferably from 20 bp to 100 bp, still preferably from 40 to 70 bp, for example of 50 bp. These sizes, in particular 50 bp, are a good compromise between too short reads that are more likely to map to more than one location in the human genome, and too long reads which raise the chance to have SNPs inside the sequence.

Sequence Mapping

The alignment of the sequences over the human genome can be carried out using any standard alignment software, for example as described in Chiu et al., 2008 or Fan et al., 2008. The human genome sequence used for the mapping is preferably a reference sequence, such as the sequences established by the NBCI (http://www.ncbi.nlm.nih.gov/assembly/2758/) or the UCSC (http://hgdownload.cse.ucsc.edu/downloads.html#human). The reference sequence is preferably February 2009 (hg19, GRCh37), also referred as hg19.

As the method according the invention comprises two sequencing steps, it also comprises two mapping steps: the mapping of the sequences obtained at the pre-sequencing step and the mapping of the sequences obtained at the massively parallel sequencing step. The two mapping steps are preferably performed in the same way, i.e. by using the same human genome sequence and/or the same alignment software.

Both mapping steps can be done over the whole sequence of the human genome, for example over the whole hg19 reference sequence.

Alternatively, the alignment can be done over only a portion of the human genome, or in other words over a partial sequence of the human genome. Generally speaking, the partial sequence of the human genome used in score calculation is obtained by masking predefined regions of the human genome. The regions to be masked can be chosen on the basis of a number of different parameters, including: a lower quality of sequencing of a region (these regions are also known as "non-well annotated regions"); the occurrence of a high number of repeats within a region; the duplication of a region within the human genome; a region with a complex architecture. The masked regions are thus preferably selected among the non-well-annotated regions of the human genome, the high copy repeat regions of the human genome, the duplicated regions of the human genome, or the regions with a complex architecture.

A region with a lower quality of sequencing or a "non-well annotated" region is for instance a region with scaffold N50 of less than 46,395,641 and/or a contig N50 of less than 38,508,932, and/or with total assembly gap length of more than 239,845,127/3,137,144,693, and/or with a genome coverage of at least 90%, preferably at least 95% (Yandell et al., 2012). Examples of non-well annotated regions are subtelomeric regions and pericentromeric regions.

Genome assemblies are composed of scaffolds and contigs. Contigs are contiguous consensus sequences that are derived from collections of overlapping reads. Scaffolds are ordered and orientated sets of contigs that are linked to one another by mate pairs of sequencing reads. A contig N50 is calculated by first ordering every contig by length from longest to shortest. Next, starting from the longest contig, the lengths of each contig are summed, until this running sum equals one-half of the total length of all contigs in the assembly. The contig N50 of the assembly is the length of the shortest contig in this list. The scaffold N50 is calculated in the same fashion but uses scaffolds rather than contigs. Scaffolds and contigs that comprise only a single read or read pair often termed 'singletons' may be excluded from these calculations, as may be contigs and scaffolds that are shorter than -800 bp.

Genome coverage refers to the percentage of the genome that is contained in the assembly based on size estimates; these are usually based on cytological techniques

A region with a complex architecture is for instance a highly variant region, for example a region with a high number of CNVs (copy number variants), and/or SNVs (single nucleotide variants) (Frazer et al., 2009). An estimate of 5% of the human genome is for instance copy number variable.

Quality control based on the amount of unique exact sequences after presequencing

Step (iv) of the method according to the invention consists in selecting a set of samples based on the quantity of unique exact sequences obtained for said samples. Step (iv) can thus consist in selecting samples having more than a minimal quantity of unique exact sequences, or, in other terms, in eliminating samples having less than a minimal quantity of unique exact sequences.

As used herein, the term "quantity" may refer to the absolute number of unique exact sequences or to a ratio. The ratio can be calculated with respect to the total number of sequence reads obtained at the presequencing step. However, the ratio is preferably calculated with respect to the number of filter-passing reads.

Filtering may consist in eliminating the sequences mapped at least partially to an adptor sequence. The number of filter passing reads is the total number of sequence reads minus the number of sequence reads mapped at least partially to an adaptor sequence.

In a preferred embodiment, step (iii) comprises selecting samples with at least 70% unique exact sequences, preferably at least 72% unique exact sequences, still preferably at least 75% or still preferably at least 77% or still preferably at least 80% of unique exact sequences with respect to the total number of sequence reads obtained at the presequencing step for said sample.

Massively parallel sequencing

Various massively parallel sequencing technologies and platforms can be employed in the present invention.

The massively parallel sequencing platform may for instance consist in a "sequencing-by-synthesis" system, such as the Illumina's HiSeq2000 platform. This platform uses a reversible terminator-based method that detects single bases as they are incorporated into growing DNA strands. The sequencing workflow can be summarized in 3 phases:

First, the preparation of the DNA library: this step has already been described and, as mentioned above, it can be carried out at an early phase of the whole process of selecting euploid appropriate reference samples, or of the diagnosis process. It is for example performed immediately after DNA extraction. During this phase, DNA molecules are ligated with adaptors at both ends. In addition, they contain primer sites that are used to amplify the library by PCR and to sequence it.

Second, the cluster generation: during this phase, DNA molecules are hybridized to oligonucleotide probes tethered on a solid surface inside a flow cell. Each DNA molecule is amplified by solid-phase bride-amplification, forming a cluster of molecules with identical sequences.

Third, the "sequencing-by-synthesis" phase. A mixture of the four nucleotides, each containing a fluorescently-labeled terminator, is introduced into the flow-cell. The fluorescently-labeled terminator is imaged as each dNTP is incorporated into the growing DNA strand, and then cleaved to allow incorporation of the next base. Since all four reversible terminator-bound dNTPs are present during each sequencing cycle, natural competition minimizes incorporation bias. Base calls are made directly from intensity signal measurements during each cycle.

In a specific embodiment, the massively parallel sequencing step consists in sequencing at least 10 millions, preferably at least 20 millions still preferably at least 30 million sequences per sample.

Alternatively or in addition, at least 6 million, preferably at least 8 million, still preferably at least 10 million, or at least 12 million or at least 14 million or at least 15 millions unique exact sequences per sample are obtained in the mapping step (step (vi)). Alternatively or in addition, a mean number of at least 12 million, preferably at least 15 million, still preferably at least 20 million unique exact sequences per sample is obtained in the mapping step (step (vi)).

The total number of sequences and/or the number of unique exact sequences obtained in the massively parallel sequencing step can also be used as a quality control criterion, in the process of selecting the samples forming the set of reference samples.

In a specific embodiment, the method for obtaining a set of euploid reference samples according to the invention comprises selecting samples with a total number of at least 10 million, preferably at least 20 million, still preferably at least 30 million sequences per sample.

Alternatively or in addition, the method for obtaining a set of euploid reference samples according to the invention comprises selecting samples with at least 6 million, preferably at least 8 million, still preferably at least 10 million, or at least 12 million or at least 14 million or at least 15 million unique exact sequences.

Alternatively or in addition, the set of reference samples has a mean total number of sequences obtained in the massively parallel sequencing step of at least 20 million, preferably at least 25 million, still preferably at least 27 million. The term "total number of sequences" may refer to the total number of non-filtered reads obtained at the sequencing step, or to the total number of filter-passing reads, in case where the sequencing platform includes a filtering. In such case, the term "total number of sequences" preferably refers to the total number of filter-passing reads.

Alternatively or in addition, the set of reference samples has a mean number of unique exact sequences of at least 12 million, preferably at least 15 million, still preferably at least 20 million.

Diagnosis method

A second aspect of the present invention consists in a method for diagnosing fetal aneuploidy from a maternal biological sample, characterized in that the sample to be diagnosed is compared to the reference set of samples obtained with the method for obtaining a set of reference samples as described above.

Briefly the workflow of this method can be summarized as follows:

- extraction of cell-free DNA from a biological sample;
- NGS sequencing of the extracted DNA molecules;
- mapping the sequences over the human genome;
- calculating the score of a chromosome or chromosomal region of interest for said sample;
- comparing said score to the set of scores obtained for the same chromosome or chromosomal region on the set of reference samples;
- diagnosing a fetal chromosomal aneuploidy or not, based on the results of the comparison.

Accordingly, in comparison to the above-described embodiment of the method for obtaining a set of reference samples, the workflow of the diagnosis method does not comprise the steps of analyzing the size distribution of the DNA molecules within each sample and selecting a set of samples based on the size distribution of the DNA molecules within said samples, and steps (iv), (v) and (vi), namely the selection based on the size distribution and the selection based on the pre-sequencing results. Of course, this does not mean that a size distribution analysis or a pre-sequencing may not be performed on a sample to be diagnosed, but these steps are optional and will normally not be practiced with a view to selecting or eliminating certain samples.

Besides these two differences, the above mentioned features and embodiments concerning a specific step in the method for selecting a set of reference samples also apply to the corresponding step in the method for diagnosing fetal aneuploidy.

Scoring algorithm

The score calculated for a given chromosome or chromosomal region is a parameter indicative of the count of unique exact sequences mapped to said chromosome or chromosomal region, for a given sample. The score can be calculated over the whole human genome sequence, or over a partial sequence of the human genome or, in other terms a sequence from which some regions have been masked.

Calculating the score only over a carefully selected portion of the human genome is a way to increase the degree of statistical confidence of the diagnosis method. Generally speaking, the partial sequence of the human genome used in score calculation is obtained by masking predefined regions of the human genome. A number of parameters can be considered for defining the regions to be masked, including a lower quality of sequencing of a region (also defined, in other terms as a non-well annotated region), the occurrence of a high number of repeat within a region, the duplication of a region within the human genome, a region with a complex architecture. The masked regions are thus preferably selected among the non-well-annotated regions of the human genome, the high copy repeat regions of the human genome, the duplicated regions of the human genome or the regions with a complex architecture.

The score for each chromosome can be calculated by dividing each chromosome into bins of a predefined length, for example 50 kb bins. The division can be carried out on a whole human genome sequence or on a partial human genome sequence, i.e. on a human genome sequence in which some regions have been masked, as explained above.

The number of unique exact sequences (UES) mapped to a given bin is then counted, thus yielding a UES count for each bin.

In a specific embodiment, the count of UES for each bin is bias-corrected, i.e. it is corrected to take into account the bias related to the sequencing process. A known bias is caused by the variation in GC distribution across the genome. As noted by Fan et al., 2010, the distribution of sequence tags across the genome is not uniform. In fact, there exists a positive correlation between the GC content of a chromosomal region, and the number of sequences mapped to said region, which explains why sequences originating from GC-rich regions are more represented within the sequence library than sequences originating from GC-poor regions. This bias can be compensated by weighting the count of UESs in each bin, for example with a weight inversely proportional to the GC content in said bin.

The median UES count value for all bins over a chromosome or chromosomal region of interest is then calculated. This value is representative of the count of UESs across the chromosome or chromosomal region, and is referred as the sequence tag density of a chromosome or chromosomal region. This median value can be calculated by using non-weighted UES counts, or by weighting each UES count with a bias-correction factor, as indicated above. In another embodiment, other values than the median value are selected for representing the UES count across a chromosome: for instance the sum of the UES counts for all bins within a chromosome.

Finally, the sequence tag density of the chromosome or chromosomal region of interest is normalized to the median sequence tag density for all chromosomes. Alternatively, it can be normalized to the median sequence tag density for all autosomes. Still alternatively, it can be normalized to the median sequence tag density for a predefined set of chromosomes. As used herein "set of chromosomes" refers to any combination of chromosomes selected from chromosome 1 to chromosome 22 and chromosome X and Y. Still alternatively, it can be normalized to the median sequence tag density for a predefined set of chromosomal regions. Still alternatively, it can be normalized to the sum of sequence tag densities for all chromosomes, or for all autosomes, or for a predefined set of chromosomes, or for a predefined set of chromosomal regions.

The normalized sequence tag density of a chromosome or chromosomal region is preferably used as a parameter indicative of the number of unique exact sequences mapped to a chromosome or chromosomal region of interest for a given sample. This parameter can be represented by other values:

- the sequence tag density of a chromosome or chromosomal region of interest;
- the number of UESs mapped to said chromosome or chromosomal region of interest;
- the number of UESs mapped to said chromosome or chromosomal region of interest normalized by the total number of UES for the sample;
- the number of UESs mapped to said chromosome or chromosomal region of interest normalized by the total number of UES mapped to a predefined set of chromosomes or chromosomal regions.

As illustrated in Figures 5 to 15, other scoring algorithms can be used for discriminating aneuploid samples from euploid samples, thus yielding other parameters indicative of the number of unique exact sequences mapped to a chromosome or chromosomal region of interest.

Preferably, the chromosome of interest is chromosome 21 and/or the fetal aneuploidy is trisomy 21. Alternatively, the chromosome of interest is chromosome 18 and/or the fetal aneuploidy is trisomy 18. Alternatively, the chromosome of interest is chromosome 13 and/or the fetal aneuploidy is trisomy 13. Alternatively, the chromosome of interest is chromosome 22 and/or the fetal aneuploidy is trisomy 22. Alternatively, the chromosome of interest is chromosome 4 and/or the fetal aneuploidy is Wolf-Hirschhorn syndrome. Alternatively, the chromosomal region of interest is a portion of chromosome 4 comprising the deleted region in Wolf-Hirschhorn syndrome. Alternatively, the chromosome of interest is chromosome 5 and/or the fetal aneuploidy is cri du chat syndrome. Alternatively, the chromosomal region of interest is a portion of chromosome 5 comprising the deleted and/or duplicated region in cri du chat syndrome and/or the fetal aneuploidy is cri du chat syndrome. Alternatively, the chromosome of interest is chromosome 19. Alternatively, the chromosome of interest is chromosome 1. Any combination of the aforementioned chromosomes or chromosomal region can also be chosen as a specific embodiment.

More preferably, the chromosome of interest is chromosome 21, chromosome 18, or chromosome 13, still preferably, the chromosome of interest is chromosome 21 or chromosome 18.

Comparison of the test sample with the set of reference samples

Whatever the test parameter selected as indicative of the number of unique exact sequences mapped to the chromosome or chromosomal region of interest for the test sample, the same parameter is calculated for each sample of the reference set of samples, thus yielding the set of reference parameters ("same parameter" means that the parameter is calculated by using the same method than used for the test sample, but applied to the sequencing data obtained on the reference sample, instead of those obtained on the test sample).

The test parameter obtained for the test sample is then compared to the set of reference parameters obtained for the reference samples.

The comparison can be done by calculating the z-score of the test sample, according to the formula:

$$\text{z-score} = (P_{test} - \text{mean}(P_{ref}))/(SD(P_{ref}))$$

wherein

- $P_{test}$ is the test parameter indicative of the number of unique exact sequences mapped to the chromosome or chromosomal region of interest, calculated from the test sample.
- Mean ($P_{ref}$) and SD($P_{ref}$) are respectively the mean and the standard deviation of the set of reference parameters indicative of the number of unique exact sequences mapped to the chromosome or chromosomal region of interest, calculated from the set of reference samples.

Preferably, the absolute value of the z-score of a sample aneuploid for the chromosome or chromosomal region of interest is above 4, still preferably above 4.4.

Preferably, the absolute value of the z-score of a sample euploid for the chromosome or chromosomal region of interest is below 4.4, still preferably below 4.

Preferably, the absolute value of the z-score of each sample of the reference set of samples is below 4.4, still preferably below 4.

As illustrated in Figures 3 and 4, the selection of an appropriate set of reference samples, by using the method according to the invention, allows discriminating trisomy 21 and trisomy 18 samples from euploid samples, with a z-score of 4.4 as cutoff value. This z-score corresponds to a prior probability of $\leq 1.1 \cdot 10^{-5}$ of generating false results by chance, which is much lower than the corresponding data in prior art.

**Examples**

Example 1

*DNA extraction from maternal blood and quality control assays*

Blood samples were collected from 100 pregnant women in the context of a prospective clinical study with pending approval by the local ethical committee. The gestational age of the mothers was $14.63 \pm 4.00$ weeks.

Two 7.5ml tubes (BD Vacutainer blood collection tubes, Beckton Dickinson, NJ USA 07417, or BCT-tubes, Streck, Inc., Omaha, NE 68128) were collected 30 minutes after invasive prenatal diagnosis. Plasma was purified as described (Chiu *et al* 2008; Fan *et al* 2008), and frozen immediately at -20°C. 2ml plasma aliquots were used for cell-free DNA extraction with the nucleospin plasma Kit (Macherely Nagel, according to the manufacturer's instructions as described below), or with a phenol-chloroform method, which was as follows.

Nucleospin plasma Kit (according to the manufacturer's instructions)

20 $\mu$l proteinase K were added to the 2 ml plasma aliquots, and the mixture was heated during 10 minutes at 37°C (without stirring). The mixture plasma-proteinase K was transferred into a 5 mL tube, then Buffer BB was added (1.5 x the plasma volume), and the tubes were mixed 3x by turning them over, and vortexed during 3 seconds. The mixture was loaded onto several columns (600 $\mu$l/column) and centrifugated at 2000g (320 rpm) during 30 seconds, then at 11000 g (9600 rpm) during 5 seconds. The columns were then washed a first time with 500$\mu$l Buffer WB and centrifugated at 11000g (9600 rpm) during 30 seconds, and a second time with 250$\mu$l Buffer WB and centrifugated at 11000g (9600 rpm) during 3 minutes. Finally, 20 $\mu$l elution buffer were added to the columns, which were then centrifugated at 11000g (9600 rpm) during 30 seconds. The resulting DNA extracts were pooled in a single 2mL tube.

Phenol-chloroform method

200 $\mu$l 10% SDS, 40 $\mu$l 0.5M EDTA and 25 $\mu$l proteinase K were added, and samples incubated for 2 hours at 58 °C. 2 ml of RT equilibrated biophenol were added, and samples agitated, and centrifuged at 4000 rpm for 10 minutes. The aqueous phase (1800 ml) was transferred to a new 5ml tube, and DNA was precipitated with 20 $\mu$l glycogen/GlycoBlue, 1/9 volume of 3M NaAc, and 0.7 volumes of ice-cold isopropanol. After vigorous vortexing, 2 ml were transferred to a new tube and centrifuged for 10 minutes in a microfuge at maximum speed. The supernatant was decanted, and the remaining volume added, and the tube centrifuged under the same conditions. The DNA pellet was first washed with 600 $\mu$l of ethanol 70%, followed by 600 $\mu$l of ether, and suspended in 20 $\mu$l of 0.5 mM Tris pH 8.2.

DNA concentration was measured with PicoGreen, and qPCR assays for THO1 and SRY were performed on samples corresponding to a male fetus. The principle of these assays is to quantify:

- Male DNA, i.e. fetal DNA, by amplifying a 137 bp sequence of the SRY gene, present on human chromosome Y;

- Total human DNA, i.e. fetal + maternal DNA, by amplifying a 162 bp sequence comprising the THO1 STR (short tandem repeat), present on human chromosome 11.

The mouse gene GALT was used as an internal control. Briefly, for each sample a master mix was prepared containing 12.5$\mu$l Absolute QPCR Mix (AB-1133/A, ABGene), 2.5 $\mu$l of a mixture of primers/probes SRY/THO1/GALT and 0.4 $\mu$l of AmpliTag Gold 5U/$\mu$l (N8080249, Applied Biosystems). 25 $\mu$l PCR mix were prepared, each containing: 5 $\mu$l of DNA sample to be amplified in $H_2O$, 5 $\mu$l Std Galt 10 copies/$\mu$l (standard sequence of GALT), 15 $\mu$l master mix.

Each series included a standard (10$\mu$l standard, 200 cell/10 $\mu$l). 50 RT-PCR cycles (95°C/15";60°C/60") were run on a RotorGene qPCR apparatus (Qiagen), with an acquisition at 60°C on the channels SRY (green), THO1 (Yellow), GALT (Red).

Table 1 shows the comparative results of nine plasma samples from pregnant women carrying male fetuses extracted in parallel with the two methods, the column- and the phenol-based method. As can be seen, the yield is significantly higher in phenol-based extractions (p=2.2·10$^{-5}$), and the phenol-based procedure yields about fivefold more DNA, and most importantly more consistent and more robust signals for SRY, i.e. for fetal DNA (p<0.05). In Table 1, the value in "cells/$\mu$l" was calculated with reference to the standard, and refers to an equivalency of the quantity of genomic DNA in terms of cell number, based on the assumption of 6 pg genomic DNA/cell.

### Example 2

Chromatin-immunoprecipitation (ChIP)-based shotgun sequencing NGS protocol

Methods

The ChIP sequencing protocol (Illumina) was performed according to instructions. 20 ng of cell-free DNA was used for library construction. 1 $\mu$l of each library, corresponding to 1/15 of the total library volume, was run on a 2100 Bioanalyzer (Agilent) for size distribution analysis and determination of peak concentration. Every fifth library was pre-sequenced on a MiSeq (Illumina). The libraries were sequenced on a HiSeq 2000 (Illumina), with single reads of 50 bp, and 50+7 cycles, thus resulting in 30·10$^6$ reads per sample, using the TruSeq SBS v3 kit according to instructions (Illumina).

On 50 samples, the two extraction protottols (column extraction and phenol/chloroform extraction) were performed in parallel, as described above. The remaining samples were extracted only by the phenol/chloroform method.

### Results

The size determination of cell-free DNA shows that after subtraction of the adaptor/barcode sequence size, the peak size is almost perfectly within the predicted size of 166 bp (Fig. 1; Lo *et al* 2010). The peak size distribution was uniform for all 91 samples analyzed, with 1-2 bp variations. The smaller sized shoulder visible on the right hand panel likely reflects fetal DNA, which has a peak size of 133-143 bp.

The phenol/chloroform extraction protocol yielded a much higher concentration of DNA molecules having a size around the peak of 166 bp, with a statistically significant difference between the column library and the phenol/chloroform library (p<10$^{-25}$; Table 2, showing the concentration of the fraction of DNA molecules with a size ranging from 156 bp to 176 bp, as measured on 50 libraries for each extraction method).

The unique exact sequences for the 30 pre-sequenced libraries (Table 3), and for the final output sequences of the 91 samples (Table 4 and Fig. 2) were between 75-80% of the filter passing reads.

Overall the median number of UESs was more than 20 million which is more than four times higher than the respective number used as a basis for the published aneuploidy test (Fan et al., 2008, Chiu et al., 2008, Stumm *et al* 2012).

Each chromosome was divided into 50 kb bins and, for each bin, the number of UESs mapped to said bin was counted. The median value of the UESs counts per bin was calculated for each chromosome, thus yielding a sequence tag density value for all autosomes.

The sequence tag density of chromosome 21 was normalized to the median value of sequence tag densities for all autosomes, thus yielding the normalized sequence tag density for chromosome 21, as shown in Fig. 3 for all 91 euploid and aneuploid samples. This value is indicative of the fraction of fetal and maternal DNA fragments issued from chromosome 21.

Samples with normal karyotypes were used to constitute a reference set that provides the basis to normalize single chromosome counts. With such a reference set, the diagnosis method according to the present invention is capable of perfectly discriminating trisomy 21 cases from non-trisomy 21 cases using a z-score of 4.4 (Fig. 3).

In a similar way, the sequence tag density of chromosome 18 was normalized to the median value of sequence tag densities for all autosomes, thus yielding the normalized sequence tag density, as shown in Figure 4 for all 91 euploid and aneuploid samples analyzed in this study.

As evident from Figure 4, the diagnosis method according to the present invention is also capable of discriminating trisomy 18 cases from non-trisomy 18 cases using a z-score of 4.4, using the same reference set of 66 euploid samples.

Overall, the method according to the invention allows a more stringent discrimination of about two orders of magnitude over first generations assays (Chiu *et al* 2008, Fan *et al* 2008, Stumm *et al* 2012) with a prior probability of $\leq 1.1 \cdot 10^{-5}$ to generate false results by chance.

Finally, another algorithm has been used for processing the data obtained from 91 samples. The results are shown in Figures 5 to 13. By using this second algorithm and a set of reference samples selected according to the method of the present invention, the diagnosis method allows discriminating trisomy 21 samples, trisomy 13 samples, trisomy 18 samples, trisomy 22 samples, microdeletion 4p samples, microdeletion-duplication 5p samples from euploid samples, with a prior probability of $\leq 1.1 \cdot 10^{-11}$ to generate false results by chance.

## References

Chen, EZ, Chiu, RWK, Sun, H, Akolekar, R, Chan, KCA, Leung, TY, Jiang, P, Zheng, YWL, Lun, FMF, Chan, LYS, Jin, Y, Go, ATJI, Lau, ET, To, WWK, Leung, WC, Tang, RYK, Au-Yeung, SKC, Lam, H, Kung, YY, Zhang, X, van Vugt, JMG, Minekawa, R, Tang, MHY, Wang, J, Oudejans, CBM, Lau, TK, Nicolaides, KH, Lo, YMD. Noninvasive Prenatal Diagnosis of Fetal Trisomy 18 and Trisomy 13 by Maternal Plasma DNA Sequencing. PLoS ONE 2011, 6(7):e21791.

Chiu RW, Chan KC, Gao Y, Lau VY, Zheng W, Leung TY, Foo CH, Xie B, Tsui NB, Lun FM, Zee BC, Lau TK, Cantor CR, Lo YM. Noninvasive prenatal diagnosis of fetal chromosomal aneuploidy by massively parallel genomic sequencing of DNA in maternal plasma. Proc Natl Acad Sci U S A. 2008 Dec 23;105(51):20458-63

Chiu, RWK, Akolekar, R, Zheng, YWL, Leung, TY, Sun, H, Chan, KCA, Lun, FMF, Go, ATJI, Lau, ET, To, WWK, Leung, WC, Tang, RYK, Au-Yeung, SKC, Lam, H, Kung, YY, Zhang, X, van Vugt, JMG, Minekawa, R, Tang, MHY, Wang, J, Oudejans, CBM, Lau, TK, Nicolaides, KH, Lo, YMD. Non-invasive prenatal assessment of trisomy 21 by multiplexed maternal plasma DNA sequencing: large scale validity study. BMJ 2011, 342:c7401-c7401.

Cooper GM, Coe BP, Girirajan S, Rosenfeld JA, Vu TH, Baker C, Williams C, Stalker H, Hamid R, Hannig V, Abdel-Hamid H, Bader P, McCracken E, Niyazov D, Leppig K, Thiese H, Hummel M, Alexander N, Gorski J, Kussmann J, Shashi V, Johnson K, Rehder C, Ballif BC, Shaffer LG, Eichler EE. A copy number variation morbidity map of developmental delay, Nat Genet. 2011 Aug 14;43(9):838-46.

Ehrich, M, Deciu, C, Zwiefelhofer, T, Tynan, JA, Cagasan, L, Tim, R, Lu, V, McCullough, R, McCarthy, E, Nygren, AOH, Dean, J, Tang, L, Hutchison, D, Lu, T, Wang, H, Angkachatchai, V, Oeth, P, Cantor, CR, Bombard, A, van den Boom, D. Noninvasive detection of fetal trisomy 21 by sequencing of DNA in maternal blood: a study in a clinical setting. American Journal of Obstetrics & Gynecology 2011, 204(3): 205.e1-205.e11

Fan HC, Blumenfeld YJ, Chitkara U, Hudgins L, Quake SR. Noninvasive diagnosis of fetal aneuploidy by shotgun sequencing DNA from maternal blood. Proc Natl Acad Sci U S A. 2008 Oct 21;105(42):16266-71

Frazer KA, Murray SS, Schork NJ, Topol EJ. Human genetic variation and its contribution to complex traits. Nat Rev Genet. 2009 Apr;10(4):241-51.

Lo YM, Lun FM, Chan KC, Tsui NB, Chong KC, Lau TK, Leung TY, Zee BC, Cantor CR, Chiu RW. Digital PCR for the molecular detection of fetal chromosomal aneuploidy. Proc Natl Acad Sci U S A. 2007 Aug 7; 104(32):13116-21.

Lo YM, Chan KC, Sun H, Chen EZ, Jiang P, Lun FM, Zheng YW, Leung TY, Lau TK, Cantor CR, Chiu RW. Maternal plasma DNA sequencing reveals the genome-wide genetic and mutational profile of the fetus. Sci Transl Med. 2010 Dec 8;2(61):61ra91

Stumm M, Entezami M, Trunk N, Beck M, Löcherbach J, Wegner RD, Hagen A, Becker R, Hofmann W. Noninvasive prenatal detection of chromosomal aneuploidies using different next generation sequencing strategies and algorithms. Prenat Diagn. 2012 Jun;32(6):569-77.

Xue, X, Teare, MD, Holen, I, Zhu, YM, Woll, PJ. Optimizing the yield and utility of circulating cell-free DNA from plasma and serum. Clinica Chimica Acta 2009, 404(2): 100-104

Yandell M, Ence D. A beginner's guide to eukaryotic genome annotation. Nat Rev Genet. 2012 Apr 18;13(5):329-42.

**Tables**

| sample | | | 266173 | 283679 | 297650 |
|---|---|---|---|---|---|
| DNA concentration measured by Picogreen | column | conc. (ng/µl) | 0.36 | 0.38 | 1.13 |
| | Phenol/Chloroform (P/C) | conc. (ng/µl) | 1.83 | 1.96 | 3.66 |
| *THO1* = total DNA | column | cells/ µl | 7.50 | 17.00 | 108.00 |
| | P/C | cells/ µl | 297.00 | 126.00 | 233.00 |
| | column | conc. (ng/µl) | 0.045 | 0.102 | 0.648 |
| | P/C | conc. (ng/µl) | 1.782 | 0.756 | 1.398 |
| | column | total DNA (ng) | 1.80 | 4.08 | 25.92 |
| | P/C | total DNA (ng) | 35.64 | 15.12 | 27.96 |
| *SRY* = fetal DNA | column | cells/ µl | 0.00 | 0.00 | 0.50 |
| | P/C | cells/ µl | 6.00 | 3.00 | 12.00 |
| | column | conc. (ng/µl) | 0.000 | 0.000 | 0.003 |
| | P/C | conc. (ng/µl) | 0.036 | 0.018 | 0.072 |
| | column | total DNA (ng) | 0.00 | 0.00 | 0.12 |
| | P/C | total DNA (ng) | 0.72 | 0.36 | 1.44 |

**Table 1:** comparison of the DNA quantity obtained by column extraction and by phenol/chloroform extraction

| sample | | | 304784 | 307020 | 313999 |
|---|---|---|---|---|---|
| DNA concentration measured by Picogreen | column | conc. (ng/ µl) | 0.40 | 0.33 | 0.40 |
| | P/C | conc. (ng/ µl) | 1.53 | 1.19 | 1.82 |
| THO1 = total DNA | column | cells/ µl | 12.00 | 2.50 | 8.50 |
| | P/C | cells/ µl | 73.00 | 29.00 | 97.00 |
| | column | conc. (ng/ µl) | 0.072 | 0.015 | 0.051 |
| | P/C | conc. (ng/ µl) | 0.438 | 0.174 | 0.582 |
| | column | total DNA (ng) | 2.88 | 0.60 | 2.04 |
| | P/C | total DNA (ng) | 8.76 | 3.48 | 11.64 |
| SRY = fetal DNA | column | cells/ µl | 2.00 | 2.00 | 2.00 |
| | P/C | cells/ µl | 4.00 | 7.00 | 1.00 |
| | column | conc. (ng/ µl) | 0.012 | 0.012 | 0.012 |
| | P/C | conc. (ng/ µl) | 0.024 | 0.042 | 0.006 |
| | column | total DNA (ng) | 0.48 | 0.48 | 0.48 |
| | P/C | total DNA (ng) | 0.48 | 0.84 | 0.12 |

**Table 1 (continued)**

| sample | | | 320395 | 320539 | 321479 |
|---|---|---|---|---|---|
| DNA concentration measured by Picogreen | column | conc. (ng/μl) | 0.48 | 0.48 | 0.40 |
| | P/C | conc. (ng/μl) | 1.83 | 1.86 | 1.38 |
| *THO1* = total DNA | column | cells/ μl | 24.50 | 20.00 | 9.50 |
| | P/C | cells/ μl | 265.00 | 191.00 | 38.00 |
| | column | conc. (ng/μl) | 0.147 | 0.120 | 0.057 |
| | P/C | conc. (ng/μl) | 1.590 | 1.146 | 0.228 |
| | column | total DNA (ng) | 5.88 | 4.80 | 2.28 |
| | P/C | total DNA (ng) | 31.80 | 22.92 | 4.56 |
| *SRY* = fetal DNA | column | cells/ μl | 3.00 | 5.50 | 0.00 |
| | P/C | cells/ μl | 9.00 | 27.00 | 0.00 |
| | column | conc. (ng/μl) | 0.018 | 0.033 | 0.000 |
| | P/C | conc. (ng/μl) | 0.054 | 0.162 | 0.000 |
| | column | total DNA (ng) | 0.72 | 1.32 | 0.00 |
| | P/C | total DNA (ng) | 1.08 | 3.24 | 0.00 |

## Table 1 (end)

**Table 2:** comparison of the DNA fraction at the peak between libraries obtained by column extraction and libraries obtained by phenol/chloroform extraction.

| | DNA concentration at the peak (156-176 bp), ng/μl | |
|---|---|---|
| Sample | Column extraction | Phenol/chloroform extraction |
| 1 | 0.444 | 1.462 |
| 2 | 0.355 | 1.736 |
| 3 | 0.465 | 1.074 |
| 4 | 0.5 | 1.078 |
| 5 | 0.465 | 1.157 |
| 6 | 0.485 | 1.276 |
| 7 | 0.449 | 1.034 |
| 8 | 0.462 | 0.998 |
| 9 | 0.436 | 1.848 |
| 10 | 0.404 | 0.892 |

(continued)

| | DNA concentration at the peak (156-176 bp), ng/μl | |
|---|---|---|
| Sample | Column extraction | Phenol/chloroform extraction |
| 11 | 0.429 | 1.039 |
| 12 | 0.45 | 0.668 |
| 13 | 0.441 | 0.762 |
| 14 | 0.444 | 0.784 |
| 15 | 0.246 | 0.768 |
| 16 | 0.366 | 0.564 |
| 17 | 0.45 | 1.662 |
| 18 | 0.372 | 1.092 |
| 19 | 0.422 | 1.346 |
| 20 | 0.417 | 0.004 |
| 21 | 0.482 | 1.35 |
| 22 | 0.462 | 0.473 |
| 23 | 0.545 | 0.95 |
| 24 | 0.438 | 0.925 |
| 25 | 0.338 | 0.844 |
| 26 | 0.37 | 1.189 |
| 27 | 0.378 | 1.363 |
| 28 | 0.459 | 1.727 |
| 29 | 0.414 | 1.478 |
| 30 | 0.465 | 0.973 |
| 31 | 0.439 | 1.115 |
| 32 | 0.464 | 0.663 |
| 33 | 0.378 | 1.828 |
| 34 | 0.363 | 1.597 |
| 35 | 0.395 | 1.193 |
| 36 | 0.344 | 1.033 |
| 37 | 0.346 | 1.313 |
| 38 | 0.461 | 1.238 |
| 39 | 0.558 | 1.211 |
| 40 | 0.375 | 1.16 |
| 41 | 0.445 | 1.712 |
| 42 | 0.501 | 1.025 |
| 43 | 0.379 | 1.311 |
| 44 | 0.388 | 1.721 |
| 45 | 0.4 | 1.541 |
| 46 | 0.378 | 1.687 |
| 47 | 0.399 | 1.136 |

(continued)

| Sample | DNA concentration at the peak (156-176 bp), ng/$\mu$l | |
|---|---|---|
| | Column extraction | Phenol/chloroform extraction |
| 48 | 0.461 | 0.818 |
| 49 | 0.487 | 1.61 |
| 50 | 0.478 | 1.049 |
| 51 | | 1.497 |
| 52 | | 1.151 |
| | | |
| mean | 0.42584 | 1.175480769 |
| standard-deviation | 0.0456592 | 0.295556213 |

**Table 3:** Number of unique exact sequences mapped from a total number of 20000 sequences obtained by pre-sequencing 30 libraries.

| Sample | Exact unique reads | Sample | Exact unique reads |
|---|---|---|---|
| 112 | 15591 | 78 | 15716 |
| 113 | 15369 | 79 | 15645 |
| 114 | 15083 | 80 | 15582 |
| 115 | 15521 | 81 | 15362 |
| 116 | 15129 | 82 | 15584 |
| 136 | 15006 | 14 | 15719 |
| 137 | 15187 | 19 | 15703 |
| 138 | 14982 | 25 | 15975 |
| 139 | 14996 | 30 | 15784 |
| 140 | 15160 | 35 | 15825 |
| 63 | 15757 | 40 | 15908 |
| 64 | 15505 | 45 | 15809 |
| 65 | 15447 | 51 | 15614 |
| 66 | 15245 | 5 | 15766 |
| 67 | 15336 | 6 | 15947 |

**Table 4:** WG-NGS sequencing results for 91 samples

| Sample | Input reads | Filtered reads | Mapped reads | Exact unique reads | Sequences mapped with one or more mismatches |
|---|---|---|---|---|---|
| 103 | 30216950 | 30206130 | 25525406 | 23058501 | 408032 |
| 104 | 41575507 | 41561036 | 35018861 | 31642410 | 832047 |
| 105 | 30365400 | 30355978 | 25546455 | 23127820 | 586418 |
| 106 | 26929445 | 26920157 | 22852752 | 20675058 | 517100 |
| 107 | 23559192 | 23552360 | 20073443 | 18170522 | 333522 |
| 108 | 35841766 | 35832591 | 30303591 | 27384117 | 564796 |

(continued)

| Sample | Input reads | Filtered reads | Mapped reads | Exact unique reads | Sequences mapped with one or more mismatches |
|---|---|---|---|---|---|
| 109 | 32571028 | 32560348 | 27595205 | 24951858 | 564542 |
| 110 | 30037865 | 30029986 | 25633058 | 23187607 | 520486 |
| 111 | 36215800 | 36206110 | 30832448 | 27871120 | 717708 |
| 112 | 20240362 | 20234989 | 17272915 | 15656244 | 308158 |
| 113 | 40910677 | 40896333 | 34571966 | 31257142 | 833972 |
| 114 | 30217103 | 30178083 | 24973149 | 22638247 | 578653 |
| 115 | 30330280 | 30321809 | 26070274 | 23612805 | 680728 |
| 116 | 26931760 | 26918081 | 22779179 | 20568770 | 452533 |
| 117 | 27360655 | 27348437 | 23236513 | 20974236 | 404360 |
| 118 | 26765065 | 26754423 | 22701971 | 20464891 | 433879 |
| 119 | 37599137 | 37589478 | 32451597 | 29356483 | 746457 |
| 120 | 24825056 | 24816163 | 21245866 | 19228130 | 470492 |
| 121 | 29537402 | 29528572 | 24710325 | 22325485 | 433134 |
| 122 | 17103858 | 17099511 | 14378837 | 13049934 | 247723 |
| 123 | 42563598 | 42552194 | 35552439 | 32136558 | 678205 |
| 124 | 43551095 | 43517872 | 33482659 | 30109044 | 630807 |
| 125 | 41990852 | 41974222 | 34640770 | 31306833 | 1000532 |
| 126 | 20165346 | 20155395 | 16655905 | 15024233 | 269142 |
| 127 | 28614212 | 28603956 | 23659793 | 21403729 | 949811 |
| 128 | 33718668 | 33708567 | 27721947 | 25014637 | 755056 |
| 129 | 35030911 | 35012344 | 28422951 | 25712044 | 869419 |
| 130 | 53813004 | 53795516 | 44175351 | 39752609 | 1360280 |
| 131 | 36645537 | 36615036 | 28239141 | 25408981 | 632266 |
| 132 | 26840630 | 26828673 | 20620904 | 18636404 | 454166 |
| 133 | 18078920 | 18073753 | 14356681 | 13056233 | 231991 |
| 134 | 19756070 | 19749327 | 15198748 | 13719465 | 260789 |
| 135 | 30444677 | 30437190 | 24117912 | 21840365 | 579143 |
| 136 | 31894048 | 31879010 | 24915781 | 22506866 | 520877 |
| 137 | 48011607 | 47995568 | 37707559 | 34048774 | 1083485 |
| 138 | 11661421 | 11657168 | 8990173 | 8153777 | 102132 |
| 139 | 12616163 | 12612823 | 9710665 | 8819368 | 171488 |
| 140 | 9920976 | 9918479 | 7728069 | 6991679 | 54117 |
| 141 | 10006824 | 10004272 | 7733082 | 6998334 | 61974 |
| 142 | 12427313 | 12424394 | 9708269 | 8784588 | 76216 |
| 143 | 27714814 | 27705165 | 19878592 | 17944936 | 372128 |
| 144 | 12886547 | 12884111 | 9206059 | 8354570 | 157030 |
| 145 | 24088740 | 24081141 | 17383294 | 15709671 | 296867 |

(continued)

| Sample | Input reads | Filtered reads | Mapped reads | Exact unique reads | Sequences mapped with one or more mismatches |
|--------|-------------|----------------|--------------|--------------------|---------------------------------------------|
| 146 | 17793195 | 17789556 | 12954854 | 11737355 | 188200 |
| 147 | 18224825 | 18217755 | 12940837 | 11664147 | 152489 |
| 148 | 33525420 | 33517783 | 24203985 | 21879456 | 435612 |
| 149 | 34901104 | 34890696 | 27315337 | 24740932 | 701564 |
| 150 | 21990971 | 21983324 | 17078337 | 15441796 | 227325 |
| 151 | 39168310 | 39155280 | 30963721 | 28011313 | 680251 |
| 152 | 26659833 | 26649486 | 20910618 | 18904908 | 394770 |
| 153 | 23922186 | 23907853 | 17946481 | 16150950 | 228865 |
| 154 | 20674249 | 20669242 | 16290179 | 14728384 | 236589 |
| 54 | 14996215 | 14990161 | 13000152 | 11786877 | 208266 |
| 55 | 13140145 | 13133309 | 11389139 | 10353263 | 193054 |
| 56 | 21107469 | 21093997 | 18114352 | 16408551 | 361513 |
| 57 | 25647495 | 25635349 | 21958581 | 19825869 | 381354 |
| 58 | 25079331 | 25066398 | 21497656 | 19427908 | 396512 |
| 59 | 21562304 | 21554485 | 18613096 | 16915587 | 506920 |
| 60 | 22897045 | 22887690 | 19602821 | 17732184 | 372554 |
| 61 | 32338889 | 32321935 | 27301126 | 24689580 | 593666 |
| 62 | 36847741 | 36828916 | 31344489 | 28369230 | 702053 |
| 63 | 35927031 | 35911885 | 31071303 | 28142827 | 827633 |
| 64 | 28003326 | 27989885 | 23929617 | 21684586 | 601376 |
| 65 | 31114673 | 31099510 | 26547388 | 24010544 | 626157 |
| 66 | 25337515 | 25318370 | 21305177 | 19262637 | 414999 |
| 67 | 23033405 | 23023505 | 19484375 | 17595988 | 560617 |
| 68 | 26289382 | 26275203 | 22188383 | 20052417 | 436272 |
| 69 | 20896294 | 20889501 | 18052042 | 16320905 | 289181 |
| 70 | 24910913 | 24902482 | 21348648 | 19292163 | 403309 |
| 71 | 31530182 | 31522332 | 27356198 | 24833875 | 1203037 |
| 72 | 36026865 | 36008135 | 30307787 | 27347037 | 590553 |
| 73 | 25684076 | 25676482 | 22067202 | 19945915 | 480520 |
| 74 | 31947959 | 31937980 | 27428733 | 24830914 | 790851 |
| 75 | 33112473 | 33097941 | 28412071 | 25679827 | 746825 |
| 76 | 24703231 | 24676714 | 20632553 | 18593626 | 352497 |
| 77 | 29564096 | 29549292 | 25361957 | 22930764 | 640474 |
| 78 | 21777623 | 21770852 | 18942463 | 17161089 | 588426 |
| 79 | 26674901 | 26665454 | 22973847 | 20841805 | 678151 |
| 80 | 22439652 | 22431977 | 19361244 | 17580935 | 966900 |
| 81 | 23817526 | 23806573 | 20208407 | 18334676 | 461005 |

(continued)

| Sample | Input reads | Filtered reads | Mapped reads | Exact unique reads | Sequences mapped with one or more mismatches |
|---|---|---|---|---|---|
| 82 | 29366328 | 29356011 | 25291368 | 22881062 | 545329 |
| 83 | 26817416 | 26808097 | 23210214 | 21019757 | 613511 |
| 84 | 28458756 | 28446919 | 24442487 | 22184749 | 827635 |
| 85 | 30556673 | 30544779 | 26388196 | 23897731 | 723278 |
| 86 | 30643037 | 30629871 | 26291378 | 23784073 | 788437 |
| 87 | 20695676 | 20686734 | 17666588 | 16048732 | 597216 |
| 88 | 24497137 | 24483389 | 20838577 | 18890408 | 482866 |
| 89 | 26833708 | 26826067 | 23124596 | 20879981 | 386833 |
| 90 | 21879935 | 21873418 | 18860169 | 17057992 | 390282 |
| 91 | 25677571 | 25663274 | 21735961 | 19613749 | 492647 |
| 92 | 23799964 | 23763339 | 19620975 | 17721272 | 502702 |
| | | | | | |
| mean | 27407366,3 | 27395889,4 | 22697321,1 | 20525553,89 | |
| SD | 8320421,37 | 8317030,09 | 6986714,897 | 6301354,914 | |

**Table 5:** karyotypes of specific samples shown in Fig. 2 to 13

| Sample ID | Karyotype |
|---|---|
| 2 | 69, XXX |
| 3 | Mos45, X(50%)/46, X, del(Y)(50%) |
| 4 | CVS/AC-LK 46, XX, CVS-Direct 47, XX, +22 |
| 26 | 46, XX |
| 40 | 47, XY, +21 |
| 44 | 47, XX, +13 |
| 45 | 47, XX, +18 |
| 55 | 47, XX, +21 |
| 56 | 47, XX, +21 |
| 61 | 47, XY, +21 |
| 63 | 47, XX, +21 |
| 68 | 47, XX, +18 |
| 69 | 46, XX, del(4p) |
| 70 | 46, XX, del(5p) |
| 71 | 47, XY, +21 |
| 72 | 47, XY, +18 |
| 83 | 47, XY, +21 |
| 85 | 47, XY, +21 |
| 88 | 47, XY, +18 |
| 89 | 47, XY, +21 |

(continued)

| Sample ID | Karyotype |
| --- | --- |
| 90 | (XY) |
| 91 | 47, XX, +13 |

**Claims**

1.  Method for obtaining a set of reference samples for the diagnosis of fetal aneuploidy from a maternal biological sample containing cell-free DNA, comprising:

    (i) extracting cell-free DNA from a set of biological samples obtained from a set of euploid pregnant women carrying a euploid fetus;
    (ii) pre-sequencing DNA of each sample;
    (iii) mapping the sequences obtained in step (ii) to the human genome;
    (iv) selecting a set of samples based on the amount of unique exact sequences mapped to the human genome in step (iii);
    (v) massively parallel sequencing DNA of each sample from said set of samples selected in step (iv);
    (vi) mapping the sequences obtained in step (v) to the human genome;
    (vii) selecting a set of reference samples based on the number of unique exact sequences mapped to the human genome in step (vi).

2.  Method according to claim 1, further comprising, between the step of extracting cell-free DNA and the step of pre-sequencing:

    - analyzing the size distribution of the DNA molecules within each sample;
    - selecting a set of samples based on the size distribution of the DNA molecules within said samples.

3.  Method according to claim 1 or 2, wherein the extraction of cell-free DNA from each sample of the set of biological samples comprises:

    - mixing said biological sample with a composition comprising chloroform and phenol;
    - extracting the aqueous phase from said mixture;
    - precipitating DNA from said aqueous phase.

4.  Method according to claim 2 to 3, wherein the step of selecting a set of samples based on the size distribution of the DNA molecules comprises selecting samples in which at least 90 wt%, preferably more than 95wt% of the DNA molecules have a size from 156 bp to 176 bp.

5.  Method according to claim 2 to 4, wherein the step of selecting a set of samples based on the size distribution of the DNA molecules comprises selecting samples with at least 0.88 ng/$\mu$l DNA molecules with a size from 156 bp to 176 bp.

6.  Method according to claim 1 to 5, wherein step (ii) comprises sequencing from 1000 to 100000 sequences within each sample.

7.  Method according to claim 1 to 6, wherein step (iv) comprises selecting samples having at least 70 % of unique exact sequences with respect to the total number of sequences obtained in step (ii).

8.  Method according to claim 1 to 7, wherein step (v) comprises sequencing at least 25 million sequences for each sample.

9.  Method according to claim 1 to 8, wherein step (vii) comprises selecting samples having more than 15 millions unique exact sequence reads.

10. Method for diagnosing fetal aneuploidy from a maternal biological test sample, comprising:

(a) extracting cell-free DNA from a maternal biological test sample obtained from a pregnant woman;

(b) massively parallel sequencing the cell-free DNA extracted from said test sample;

(c) mapping the sequences obtained in step (b) to the human genome;

(d) calculating a test parameter indicative of the number of unique exact sequences mapped to a chromosome or chromosomal region of interest;

(e) calculating a set of reference parameters, wherein each reference parameter is indicative of the number of unique exact sequences mapped to a chromosome or chromosomal region of interest for a sample of a set of reference samples as obtained in claims 1 to 8;

(f) comparing said test parameter calculated in step (d) with said set of reference parameters calculated in step (e);

(g) based on the comparison, diagnosing a fetal aneuploidy.

11. Method according to claim 10, wherein the extraction of cell-free DNA from the maternal biological test sample comprises:

- mixing said biological sample with a composition comprising chloroform and phenol;
- extracting the aqueous phase from said mixture;
- precipitating DNA from said aqueous phase.

12. Method according to claim 10 or 11, wherein said test parameter is the unique sequence tag density of the chromosome or chromosomal region of interest normalized to the median unique exact sequence tag density of all autosomes.

13. Method according to claim 10 to 12, wherein the comparison in step (f) is made through calculation of the z-score of said test parameter with respect to the set of reference parameters.

14. Method according to claim 10 to 13, wherein the chromosome of interest is chromosome 21, chromosome 18 or chromosome 13.

15. Method according to claim 13, wherein the chromosome of interest is chromosome 21, and the z-score of a trisomy 21 sample is at least 4.4 while the absolute value of the z-score of a sample euploid for chromosome 21 is less than 4.4.

**Patentansprüche**

1. Verfahren zum Erhalten eines Satzes von Referenzproben aus einer mütterlichen biologischen Probe, die zellfreie DNA enthält, für die Diagnose von Fötusaneuploidie wobei das Verfahren Folgendes umfasst:

(i) Extrahieren von zellfreier DNA von einem Satz von biologischen Proben, der von einem Satz von euploiden schwangeren Frauen, die einen euploiden Fötus tragen, erhalten wurden;

(ii) Vorsequenzieren der DNA von jeder Probe;

(iii) Kartieren der in Schritt (ii) erhaltenen Sequenzen auf das menschliche Genom;

(iv) Auswählen eines Satzes von Proben auf Grundlage der Menge an einzigartigen exakten Sequenzen, die in Schritt (iii) auf das menschliche Genom kartiert worden sind;

(v) massives paralleles Sequenzieren von DNA von jeder Probe des in Schritt (iv) ausgewählten Satzes von Proben;

(vi) Kartieren der in Schritt (v) erhaltenen Sequenzen auf das menschliche Genom;

(vii) Auswählen eines Satzes von Referenzproben auf Grundlage der Anzahl von einzigartigen exakten Sequenzen, die in Schritt (vi) auf das menschliche Genom kartiert worden sind.

2. Verfahren nach Anspruch 1, das zwischen dem Schritt des Extrahierens von zellfreier DNA und dem Schritt des Vorsequenzierens weiterhin Folgendes umfasst:

- Analysieren der Größenverteilung der DNA-Moleküle innerhalb jeder Probe;
- Auswählen eines Satzes von Proben auf Grundlage der Größenverteilung der DNA-Moleküle innerhalb dieser Proben.

3. Verfahren nach Anspruch 1 oder 2, wobei das Extrahieren von zellfreier DNA aus jeder Probe des Satzes von biologischen Proben Folgendes umfasst:

- Mischen der biologischen Probe mit einer Zusammensetzung, die Chloroform und Phenol umfasst;
- Extrahieren der wässrigen Phase aus der Mischung;
- Ausfällen von DNA aus der wässrigen Phase.

4. Verfahren nach Anspruch 2 bis 3, wobei der Schritt des Auswählens eines Satzes von Proben auf Grundlage der Größenverteilung der DNA-Moleküle das Auswählen von Proben umfasst, in denen mindestens 90 Gew.-%, vorzugweise mehr als 95 Gew.-%, der DNA-Moleküle eine Größe von 156 bp bis 176 bp aufweisen.

5. Verfahren nach Anspruch 2 bis 4, wobei der Schritt des Auswählens eines Satzes von Proben auf Grundlage der Größenverteilung der DNA-Moleküle das Auswählen von Proben mit mindestens 0,88 ng/µl DNA-Molekülen mit einer Größe von 156 bp bis 176 bp umfasst.

6. Verfahren nach Anspruch 1 bis 5, wobei Schritt (ii) das Sequenzieren von 1000 bis 100000 Sequenzen innerhalb jeder Probe umfasst.

7. Verfahren nach Anspruch 1 bis 6, wobei Schritt (iv) das Auswählen von Proben mit mindestens 70% einzigartigen exakten Sequenzen in Bezug auf die Gesamtzahl der in Schritt (ii) erhaltenen Sequenzen umfasst.

8. Verfahren nach Anspruch 1 bis 7, wobei Schritt (v) das Sequenzieren von mindestens 25 Millionen Sequenzen pro Probe umfasst.

9. Verfahren nach Anspruch 1 bis 8, wobei Schritt (vii) das Auswählen von Proben mit mehr als 15 Millionen einzigartigen exakten Sequenzabschnitten umfasst.

10. Verfahren zum Diagnostizieren von Fötusaneuploidie von einer mütterlichen biologischen Testprobe, das Folgendes umfasst:

(a) Extrahieren von zellfreier DNA aus einer von einer schwangeren Frau erhaltenen mütterlichen biologischen Testprobe;
(b) massives paralleles Sequenzieren der aus der Testprobe extrahierten zellfreien DNA;
(c) Kartieren der in Schritt (b) erhaltenen Sequenzen auf das menschliche Genom;
(d) Berechnen eines Testparameters, der bezeichnend ist für die Anzahl der einzigartigen exakten Sequenzen, die auf ein Chromosom oder eine Chromosomenregion von Interesse kartiert sind;
(e) Berechnen eines Satzes von Referenzparametern, wobei jeder Referenzparameter bezeichnend ist für die Anzahl von einzigartigen exakten Sequenzen, die auf ein Chromosom oder eine Chromosomenregion von Interesse kartiert sind, für eine Probe eines gemäss Ansprüchen 1 bis 8 erhaltenen Satzes von Referenzproben;
(f) Vergleichen des in Schritt (d) berechneten Testparameters mit dem in Schritt (e) berechneten Satz von Referenzparametern;
(g) Diagnostizieren einer Fötusaneuploidie auf Grundlage des Vergleichs.

11. Verfahren nach Anspruch 10, wobei das Extrahieren von zellfreier DNA aus jeder Probe des Satzes von biologischen Proben Folgendes umfasst:

- Mischen der biologischen Probe mit einer Zusammensetzung, die Chloroform und Phenol umfasst;
- Extrahieren der wässrigen Phase aus der Mischung;
- Ausfällen von DNA aus der wässrigen Phase.

12. Verfahren nach Anspruch 10 oder 11, wobei es sich bei dem Testparameter um die einzigartige Sequenz-Tag-Dichte des Chromosoms oder der Chromosomenregion von Interesse, normalisiert auf die durchschnittliche einzigartige exakte Sequenz-Tag-Dichte aller Autosomen, handelt.

13. Verfahren nach Anspruch 10 bis 12, wobei der Vergleich in Schritt (f) durch Berechnen des z-Score des Testparameters in Bezug auf den Satz von Referenzparametern erfolgt.

14. Verfahren nach Anspruch 10 bis 13, wobei es sich bei dem Chromosom von Interesse um Chromosom 21, Chromosom 18 oder Chromosom 13 handelt.

15. Verfahren nach Anspruch 13, wobei es sich bei dem Chromosom von Interesse um Chromosom 21 handelt und der z-Score einer Trisomie-21-Probe mindestens 4,4 beträgt, während der absolute Wert des z-Score einer Probe, die für Chromosom 21 euploid ist, weniger als 4,4 beträgt.

**Revendications**

1. Procédé pour l'obtention d'un ensemble d'échantillons de référence pour le diagnostic d'une aneuploïdie foetale à partir d'un échantillon biologique maternel contenant un ADN acellulaire, comprenant :

    (i) l'extraction de l'ADN acellulaire d'un ensemble d'échantillons biologiques obtenu d'un ensemble de femmes enceintes euploïdes portant un foetus euploïde ;
    (ii) le pré-séquençage de l'ADN de chaque échantillon ;
    (iii) la cartographie des séquences obtenues dans l'étape (ii) sur le génome humain ;
    (iv) la sélection d'un ensemble d'échantillons sur la base de la quantité des séquences exactes uniques cartographiées sur le génome humain dans l'étape (iii) ;
    (v) le séquençage massivement parallèle de l'ADN de chaque échantillon, provenant dudit ensemble d'échantillons, sélectionné dans l'étape (iv) ;
    (vi) la cartographie des séquences obtenues dans l'étape (v) sur le génome humain ;
    (vii) la sélection d'un ensemble d'échantillons de référence sur la base du nombre de séquences exactes uniques cartographiées sur le génome humain dans l'étape (vi).

2. Procédé selon la revendication 1, qui comprend en outre, entre l'étape d'extraction de l'ADN acellulaire et l'étape de pré-séquençage :

    - l'analyse de la distribution des tailles des molécules d'ADN dans chaque échantillon ;
    - la sélection d'un ensemble d'échantillons sur la base de la distribution des tailles des molécules d'ADN dans lesdits échantillons.

3. Procédé selon la revendication 1 ou 2, dans lequel l'extraction de l'ADN acellulaire de chaque échantillon de l'ensemble d'échantillons biologiques comprend :

    - le mélange dudit échantillon biologique avec une composition comprenant du chloroforme et du phénol ;
    - l'extraction de la phase aqueuse dudit mélange ;
    - la précipitation de l'ADN de ladite phase aqueuse.

4. Procédé selon l'une des revendications 2 ou 3, dans lequel l'étape de sélection d'un ensemble d'échantillons sur la base de la distribution des tailles des molécules d'ADN comprend la sélection d'échantillons dans lesquels au moins 90 % en poids, de préférence plus de 95 % en poids des molécules d'ADN ont une taille de 156 à 176 pb.

5. Procédé selon l'une des revendications 2 à 4, dans lequel l'étape de sélection d'un ensemble d'échantillons sur la base de la distribution des tailles des molécules d'ADN comprend la sélection d'échantillons ayant au moins 0,88 ng/$\mu$l de molécules d'ADN ayant une taille de 156 à 176 pb.

6. Procédé selon l'une des revendications 1 à 5, dans lequel l'étape (ii) comprend le séquençage de 1000 à 100 000 séquences dans chaque échantillon.

7. Procédé selon l'une des revendications 1 à 6, dans lequel l'étape (iv) comprend la sélection d'échantillons ayant au moins 70 % de séquences exactes uniques par rapport au nombre total de séquences obtenues dans l'étape (ii).

8. Procédé selon l'une des revendications 1 à 7, dans lequel l'étape (v) comprend le séquençage d'au moins 25 millions de séquences pour chaque échantillon.

9. Procédé selon l'une des revendications 1 à 8, dans lequel l'étape (vii) comprend la sélection d'échantillons ayant plus de 15 millions de lectures de séquences exactes uniques.

10. Procédé de diagnostic d'une aneuploïdie foetale d'un échantillon d'essai biologique maternel, comprenant :

    (a) l'extraction d'un ADN acellulaire d'un échantillon d'essai biologique maternel obtenu d'une femme enceinte ;

(b) le séquençage massivement parallèle de l'ADN acellulaire extrait dudit échantillon d'essai ;

(c) la cartographie des séquences obtenues dans l'étape (d) sur le génome humain ;

(d) le calcul d'un paramètre d'essai indicatif du nombre de séquences exactes uniques cartographiées sur un chromosome ou une région chromosomique d'intérêt ;

(e) le calcul d'un ensemble de paramètres de référence, chaque paramètre de référence étant indicatif du nombre de séquences exactes uniques cartographiées sur un chromosome ou une région chromosomique d'intérêt, pour un échantillon d'un ensemble d'échantillons de référence tel qu'obtenu dans les revendications 1 à 8 ;

(f) la comparaison dudit paramètre d'essai calculé dans l'étape (d) avec ledit ensemble de paramètres de référence calculé dans l'étape (e) ;

(g) sur la base de la comparaison, le diagnostic d'une aneuploïdie foetale.

11. Procédé selon la revendication 10, dans lequel l'extraction de l'ADN acellulaire de l'échantillon d'essai biologique maternel comprend :

- le mélange dudit échantillon biologique avec une composition comprenant du chloroforme et du phénol ;
- l'extraction de la phase aqueuse dudit mélange ;
- la précipitation de l'ADN de ladite phase aqueuse.

12. Procédé selon la revendication 10 ou 11, dans lequel ledit paramètre d'essai est la densité d'étiquettes de séquences uniques du chromosome ou de la région chromosomique d'intérêt, normalisée à la densité médiane d'étiquettes de séquences exactes de tous les autosomes.

13. Procédé selon l'une des revendications 10 à 12, dans lequel la comparaison dans l'étape (f) est effectuée par calcul du score z dudit paramètre d'essai par rapport à l'ensemble de paramètres de référence.

14. Procédé selon l'une des revendications 10 à 13, dans lequel le chromosome d'intérêt est le chromosome 21, le chromosome 18 ou le chromosome 13.

15. Procédé selon la revendication 13, dans lequel le chromosome d'intérêt est le chromosome 21, et le score z d'un échantillon de trisomie 21 est d'au moins 4,4, tandis que la valeur absolue du score z d'un échantillon euploïde pour le chromosome 21 est inférieure à 4,4.

Fig. 1

Fig. 2

Fig. 3

Fig. 4

Fig. 5

EP 2 728 014 B1

Fig. 6

1/100'000'000'000

Chromosome 1

Fig. 7

1/100'000'000'000

Chromosome 19

Fig. 8

Chromosome 13

1/100'000'000'000

Fig. 9

Chromosome 18

1/100'000'000'000

Fig. 10

Fig. 11

Fig. 12

Chromosome 4

1/100'000'000'000

Fig. 13

Chromosome 5

1/100'000'000'000

Fig. 15

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2012270739 A

- WO 2011090556 A

### Non-patent literature cited in the description

- **CHEN, EZ ; CHIU, RWK ; SUN, H ; AKOLEKAR, R ; CHAN, KCA ; LEUNG, TY ; JIANG, P ; ZHENG, YWL ; LUN, FMF ; CHAN, LYS.** Noninvasive Prenatal Diagnosis of Fetal Trisomy 18 and Trisomy 13 by Maternal Plasma DNA Sequencing. *PLoS ONE,* 2011, vol. 6 (7), e21791

- **CHIU RW ; CHAN KC ; GAO Y ; LAU VY ; ZHENG W ; LEUNG TY ; FOO CH ; XIE B ; TSUI NB ; LUN FM.** Noninvasive prenatal diagnosis of fetal chromosomal aneuploidy by massively parallel genomic sequencing of DNA in maternal plasma. *Proc Natl Acad Sci U S A.,* 23 December 2008, vol. 105 (51), 20458-63

- **CHIU, RWK ; AKOLEKAR, R ; ZHENG, YWL ; LEUNG, TY ; SUN, H ; CHAN, KCA ; LUN, FMF ; GO, ATJI ; LAU, ET ; TO, WWK.** Non-invasive prenatal assessment of trisomy 21 by multiplexed maternal plasma DNA sequencing: large scale validity study. *BMJ,* 2011, vol. 342, c7401-c7401

- **COOPER GM ; COE BP ; GIRIRAJAN S ; ROSENFELD JA ; VU TH ; BAKER C ; WILLIAMS C ; STALKER H ; HAMID R ; HANNIG V.** A copy number variation morbidity map of developmental delay. *Nat Genet.,* 14 August 2011, vol. 43 (9), 838-46

- **EHRICH, M ; DECIU, C ; ZWIEFELHOFER, T ; TYNAN, JA ; CAGASAN, L ; TIM, R ; LU, V ; MCCULLOUGH, R ; MCCARTHY, E ; NYGREN, AOH.** Noninvasive detection of fetal trisomy 21 by sequencing of DNA in maternal blood: a study in a clinical setting. *American Journal of Obstetrics & Gynecology,* 2011, vol. 204 (3), 205.e1-205.e11

- **FAN HC ; BLUMENFELD YJ ; CHITKARA U ; HUDGINS L ; QUAKE SR.** Noninvasive diagnosis of fetal aneuploidy by shotgun sequencing DNA from maternal blood. *Proc Natl Acad Sci U S A.,* 21 October 2008, vol. 105 (42), 16266-71

- **FRAZER KA ; MURRAY SS ; SCHORK NJ ; TOPOL EJ.** Human genetic variation and its contribution to complex traits. *Nat Rev Genet.,* April 2009, vol. 10 (4), 241-51

- **LO YM ; LUN FM ; CHAN KC ; TSUI NB ; CHONG KC ; LAU TK ; LEUNG TY ; ZEE BC ; CANTOR CR ; CHIU RW.** Digital PCR for the molecular detection of fetal chromosomal aneuploidy. *Proc Natl Acad Sci U S A.,* 07 August 2007, vol. 104 (32), 13116-21

- **LO YM ; CHAN KC ; SUN H ; CHEN EZ ; JIANG P ; LUN FM ; ZHENG YW ; LEUNG TY ; LAU TK ; CANTOR CR.** Maternal plasma DNA sequencing reveals the genome-wide genetic and mutational profile of the fetus. *Sci Transl Med.,* 08 December 2010, vol. 2 (61), 61ra91

- **STUMM M ; ENTEZAMI M ; TRUNK N ; BECK M ; LÖCHERBACH J ; WEGNER RD ; HAGEN A ; BECKER R ; HOFMANN W.** Noninvasive prenatal detection of chromosomal aneuploidies using different next generation sequencing strategies and algorithms. *Prenat Diagn.,* June 2012, vol. 32 (6), 569-77

- **XUE, X ; TEARE, MD ; HOLEN, I ; ZHU, YM ; WOLL, PJ.** Optimizing the yield and utility of circulating cell-free DNA from plasma and serum. *Clinica Chimica Acta,* 2009, vol. 404 (2), 100-104

- **YANDELL M ; ENCE D.** A beginner's guide to eukaryotic genome annotation. *Nat Rev Genet.,* 18 April 2012, vol. 13 (5), 329-42